# EUROPEAN PATENT SPECIFICATION

(11) **EP 3 571 700 B1**
(45) Date of publication and mention of the grant of the patent: **02.08.2023**
(21) Application number: 18711395.6
(22) Date of filing: 22.01.2018
(51) Int. Cl.: G16H 50/70, G16H 50/20, G16H 10/60, G16H 50/30, G16H 70/40

(54) **METHOD AND SYSTEM FOR PREDICTING REFRACTORY EPILEPSY STATUS**
VERFAHREN UND SYSTEM ZUR VORHERSAGE DES REFRAKTÄREN EPILEPSIEZUSTANDES
PROCÉDÉ ET SYSTÈME DE PRÉDICTION D'ÉTAT D'ÉPILEPSIE RÉFRACTAIRE

(30) Priority: 23.01.2017 US 201715412806
(43) Date of publication of application: 27.11.2019
(73) Proprietor: UCB Biopharma SRL, 1070 Brussels (BE)
(72) Inventor: MALHOTRA, Kunal, Atlanta, GA 30332 (US); AN, Sungtae, Atlanta, GA 30332 (US); SUN, Jimeng, Atlanta, GA 30332 (US); CHOI, Myung, Atlanta, GA 30308 (US); DILLEY, Cynthia, Smyrna, GA 30080 (US); CLARK, Chris, Smyrna, GA 30080 (US); ROBERTSON, Joseph, Smyrna, GA 30080 (US); HAN-BURGESS, Edward, Smyrna, GA 30080 (US)
(74) Representative: UCB Intellectual Property
(86) International application number: PCT/IB2018/000089
(87) International publication number: WO 2018/134682

(56) References cited:
- RAMOS-LIZANA J ET AL: "Early prediction of refractory epilepsy in childhood", SEIZURE, BAILLIERE TINDALL, LONDON, GB, vol. 18, no. 6, 1 July 2009 (2009-07-01), pages 412-416, XP026148234, ISSN: 1059-1311, DOI: 10.1016/J.SEIZURE.2009.02.006 [retrieved on 2009-03-26]
- ORRIN DEVINSKY ET AL: "Changing the approach to treatment choice in epilepsy using big data", EPILEPSY AND BEHAVIOR, vol. 56, 1 March 2016 (2016-03-01), pages 32-37, XP055474151, US ISSN: 1525-5050, DOI: 10.1016/j.yebeh.2015.12.039
- M. J. BRODIE: "Diagnosing and predicting refractory epilepsy", ACTA NEUROLOGICA SCANDINAVICA., vol. 112, no. s181, 1 December 2005 (2005-12-01), pages 36-39, XP055475769, DK ISSN: 0001-6314, DOI: 10.1111/j.1600-0404.2005.00507.x
- PATRICK KWAN ET AL: "Early Identification of Refractory Epilepsy", NEW ENGLAND JOURNAL OF MEDICINE, THE - NEJM -, vol. 342, no. 5, 3 February 2000 (2000-02-03), pages 314-319, XP055475909, US ISSN: 0028-4793, DOI: 10.1056/NEJM200002033420503
- Frank Semah ET AL: "Can we predict refractory epilepsy at the time of diagnosis?", Epileptic Disord, 1 September 2005 (2005-09-01), pages S10-S13, XP055475926, United States Retrieved from the Internet: URL:http://www.jle.com/en/revues/epd/e-doc s/can_we_predict_refractory_epilepsy_at_th e_time_of_diagnosis__266564/article.phtml? tab=texte [retrieved on 2018-05-16]

## Description

The present disclosure relates generally to a method of predicting patient treatment refractoriness and more specifically to a method of predicting patient treatment refractoriness for epilepsy patients.

### BACKGROUND

Epilepsy is one of the most common serious neurological disorders and one of the major causes of concern affecting an estimated 50 million people worldwide. The overall annual incidence of epilepsy cases falls between 50 to 70 cases per 100,000 in industrialized countries all the way up to 190 per 100,000 in developing countries. The consequences faced by patients suffering from this disease especially the ones who are prescribed multiple treatment regimens are debilitating considering the resulting effect on their health and quality of life. According to one prediction, approximately 50% of the epilepsy patients achieve seizure control with the first anti-epilepsy drug (AED) prescribed to them, whereas approximately another 20% spend at least 2 to 5 years to find the appropriate AED regimen. The cohort of patients which are a major cause of concern are the approximately 30% of patients which do not seem to get relief from any of the existing AEDs currently in the market.

In epilepsy treatment, clinicians can choose from a pool of twenty-five different AEDs when deciding treatment regimens for patients, which is almost twice the number of drugs that were available a decade ago. Although patients are prescribed a single drug which helps in reduction of seizure frequency, there are times when patients are prescribed more than one drug depending on the type of epilepsy, patient's age, side effects of medications and other comorbidities.

In the field of epilepsy, patients are broadly characterized into refractory and nonrefractory subtypes based on the response to antiepileptic medication. Nonrefractory patients can be defined as patients which have reduced seizure frequency with the first antiepileptic drug or with few drugs prescribed, whereas refractory patients fail to get respite from seizures even with multiple treatment regimens. More specifically, nonrefractory patients are defined by the International League Against Epilepsy as patients in which "adequate trial of two tolerated, appropriately chosen and used AED schedules (whether as monotherapies or in combination) to achieve sustained seizure freedom." Refractory patients, also known as drug resistant patients, represent about 30% of the epileptic population and bear the greatest economic and psychosocial burdens. Furthermore, it has been shown that early identification of refractory patients can aid in careful management of the same, thus making it indispensable to identify the potential for patients to progress to a refractory status as soon as possible. Such management can include triage to specialists, fast track pathway to trial of new drugs and earlier surgery recommendation.

Clinical studies exist which have attempted to correlate clinical indicators to the refractory nature of patients, such as Kwan et al., "Early Identification of Refractory Epilepsy," N Engl J Med 2000; 342:314-319, February 3, 2000, and predict suitable anti-epilepsy drugs (AEDs), such as Devinsky et al., "Changing the approach to treatment choice in epilepsy using big data," Epilepsy & Behavior, Jan. 29, 2016, but there still exists a huge gap in understanding the factors which may drive the failure of a particular drug amongst refractory patients.

In the last decade, machine learning has seen the rise of neural networks with many layers. These are commonly referred to as deep neural networks (DNN). Recurrent Neural Network (RNN) is an important class of DNN. A unique aspect of RNN is the folding out in time operation, where each time-step corresponds to a layer in a feedforward network. RNN's show great performance in modeling variable length sequential data, particularly those with gated activation units such as Long Short-Term Memory (LSTM), as described in Hochreiter et al., "Long short-term memory," Neural Comput. 9, 1735-1780 (1997), and Gated Recurrent Units (GRU), as described in Chung et al., "Empirical evaluation of gated recurrent neural networks on sequence modeling," arXiv preprint arXiv:1412, 3555 (2014). RNNs have achieved state-of-the-art results in machine translation, as described in Cho et al., "Learning Phrase Representations using RNN Encoder-Decoder for Statistical Machine Translation arXiv [cs.CL] (2014), speech recognition, as described in Graves et al., "Speech Recognition with Deep Recurrent Neural Networks" arXiv [cs.NE](2013), language modeling, as described in Mikolov et al., INTER SPEECH 2010, 11th Annual Conference of the International Speech Communication Association, Makuhari, Chiba, Japan, September 26-30, 2010, (2010), pp. 1045-1048, and image caption generation, as described in Xu et al., "Show, Attend and Tell: Neural Image Caption Generation with Visual Attention," arXiv [cs.LG] (2015), due to their ability to capture long-term dependencies. RNNs have also been applied to several clinical applications recently. Lipton et al used LSTM RNN to recognize patterns in multivariate time series of clinical measurements gathered from an intensive care unit (ICU), as described in Lipton et al., "Learning to Diagnose with LSTM Recurrent Neural Networks," arXiv [cs.LG] (2015). Choi et al developed an application of RNN with GRU to jointly forecast the future disease diagnosis and medication prescription along with their timing as continuous multi-label predictions, as described in Choi et al., "Doctor AI: Predicting Clinical Events via Recurrent Neural Networks," arXiv [cs.LG] (2015). Ramos-Lizana et al., "Early prediction of refractory epilepsy in childhood", Seizure, vol 18. pp 412-416, discloses a method for identifying early predictors (variables) of refractory epilepsy. However, This document only analyzes statistical significance of different features for the outcome (refractory or not). This document represents the closest prior art.

However, these techniques are not applicable to predicting refractoriness, as refractoriness is determined by monitoring the seizure frequency over time and there is no available data source providing seizure information, as seizures are not captured in the claims data. Additionally, such techniques are not implementable into EMR systems such that they are interoperable with different coding system and can pull EMR data from EMRs and run them through a predictive model to generate refractoriness predictions.

### SUMMARY OF THE INVENTION

The present invention provides systems and methods that can predict epilepsy refractoriness based on EMR data and are implementable into EMR systems such that they are interoperable with different coding system and can pull EMR data from EMRs and run the EMR data through a predictive model to generate refractoriness predictions.

A method of building a machine learning pipeline for predicting refractoriness of epilepsy patients is provided. The method includes providing electronic health records data; constructing a patient cohort from the electronic health records data by selecting patients based on failure of at least one anti-epilepsy drug; constructing a set features found in or derived from the electronic health records data; electronically processing the patient cohort to identify a subset of the features that are predictive for refractoriness for inclusion in a predictive model configured for classifying patients as refractory or non-refractory; and training the predictive computerized model to classify the patients having at least one anti-epilepsy drug failure based on likelihood of becoming refractory.

A computer platform for generating epilepsy refractoriness predictions is also provided. The computer platform includes a client configured for interfacing with a data interface server, the data interface server configured to request formatted electronic medical records data for a patient from an electronic medical records database; a feature mapping tool configured for mapping features from the formatted electronic medical records data into a further format; a model deployment tool configured for deploying a pretrained epilepsy refractoriness prediction model; an epilepsy refractoriness prediction generator configured for generating an epilepsy refractoriness prediction for the patient by running the mapped features through the pretrained epilepsy refractoriness prediction model, the epilepsy refractoriness prediction generator including an epilepsy refractoriness prediction application configured for generating a display representing the epilepsy refractoriness prediction.

A computerized method for generating epilepsy refractoriness predictions is also provided. The method includes providing a pretrained epilepsy refractoriness prediction model; requesting, via a client, formatted electronic medical records data for a patient from an electronic medical records database; mapping features from the formatted electronic medical records data into a further format; generating an epilepsy refractoriness prediction for the patient by running the mapped features through the pretrained epilepsy refractoriness prediction model; and generating a display representing the epilepsy refractoriness prediction.

In further embodiments, computer readable media are provided which have stored thereon, computer executable process steps operable to control a computer to perform the method for generating epilepsy refractoriness predictions is also provided.

### BRIEF DESCRIPTION OF THE DRAWINGS

The present invention is described below by reference to the following drawings, in which:
Fig. 1 shows an illustration of an exemplary directed graph representing prescription information for two different patients in accordance with an embodiment of the present invention;
Fig. 2 schematically shows a flow chart of a method of generating a predictive model in accordance with an embodiment of the present invention;
Figs. 3a to 3e graphically illustrate the elimination of certain clinically insignificant gaps between consecutive prescriptions of the same drug for each patient in accordance with an embodiment of the present invention;
Fig. 4 shows a flowchart for eliminating the gaps as depicted in Figs. 3a to 3e;
Fig. 5 shows a flowchart for constructing an initial cohort for the model in accordance with an embodiment of the present invention;
Fig. 6 graphically depicts an index date defining a dividing point in the timeline of a patient in accordance with an embodiment of the present invention;
Fig. 7 graphically illustrates exemplary AED failure results;
Fig. 8 shows a flowchart for selecting features for the predictive model in accordance with an embodiment of the present invention;
Fig. 9 illustrates a predictive model in accordance with an embodiment of the present invention;
Fig. 10 shows an example of lines representing predictive models having three different classifiers on a graph of AUC versus the percentile of features included in the predictive model.
Fig. 11 shows a flowchart for training the predictive model in accordance with an embodiment of the present invention;
Fig. 12 shows a graphical illustrates an example of Pre/Post-index data availability criteria in accordance with an embodiment of the present invention;
Fig. 13 shows a data processing pipeline for constructing a plurality of different training sets in accordance with an embodiment of the present invention;
Fig. 14 shows a graphical depiction of training using an RNN including a pre-trained embedding layer and an RNN including a randomly initialized embedding layer;
Figs 15a to 15c illustrate sunblast visualizations of AED prescription patterns;
Fig. 16 illustrates a computer network in accordance with an embodiment of the present invention for deploying the predictive model;
Fig. 17 shows a flow chart illustrating a computerized method of generating and outputting of epilepsy refractoriness predictions in response to inputs of patient EMR data; and
Figs. 18a to 18d show a graphical user interface in accordance with an embodiment of the present invention.

### DETAILED DESCRIPTION

In order to provide insight into epilepsy, the present disclosure addresses the problem of epilepsy patient refractoriness by using sequential pattern mining techniques to generate frequent treatment pathways for epilepsy patients across different age groups and types of epilepsy and perform an exploratory analysis of the variations that exist in care given out to epilepsy patients. An extensive analysis of the severity of comorbidities and other medical conditions between consecutive failures in a frequent treatment pathway helps in discovering reasons driving the failure of AEDs.

Sequential pattern mining can be used for constructing epilepsy treatment pathways, which involves developing popular treatment pathways consisting of AED prescriptions as monotherapy or a polytherapy, to provide insight into how AEDs are prescribed in practice across age groups and across different types of epilepsy. These pathways are based on patterns which exist in the dataset consisting of more than one AED failing in a particular sequence more commonly than the others. This analysis is performed to explore AED failure patterns across different age groups and types of epilepsy to assess the variation in treatment routes. Frequent routes of treatment are visualized using sequential pattern mining to mine patterns from data occurring above a predetermined threshold.

The classical approach to sequential pattern mining generates patterns which are ordered sets of events which may have intermediate events occurring between them. For example, patterns consisting of diagnoses 'Depression' followed by `Mental retardation' may not necessarily mean all patients suffered from mental retardation immediately after depression.

To accomplish this, in one preferred embodiment, constraint based sequential mining, is used to restrict the extraction of frequent treatment patterns consisting of consecutive occurrence of AEDs following a pattern in a minimum threshold number of patients. Medically relevant constraints have been incorporated such as `Exact-order', which restricts the events in the pattern to occur immediately after one another. Another constraint is the temporal overlap constraint which takes into considerations overlapping events when extracting sequential patterns.

Constraint based sequential mining is described in Malhotra et al., "Constraint Based Temporal Event Sequence Mining for Glioblastoma Survival Prediction." Journal of biomedical informatics 61, page 267-275 (2016), with respect to glioblastoma survival prediction. Malhotra et al. added a constraint that the patterns which are generated consists of events which immediately follow each other, in contrast to the present embodiment, where each event is time stamped and multiple events can include the same time stamp. Also in contrast to Malhotra et al., one embodiment of the present invention analyzes all possible combination of events to check if they satisfy the minimum support level, i.e., a minimum number of patients which follow that particular pattern.

In the present method, the constraint based sequential mining approach represents the treatment data as a directed graph with patients and AEDs as nodes and edges between the AED nodes signifying the sequence of prescribed drugs. The graph by default generates patterns consisting of monotherapies and is customizable to handle polytherapies. The generation of a treatment pattern from this graph is guided by the number of patients prescribed that particular pattern. For every pattern that exists in the graph, the number of patients prescribed that particular pattern is calculated.

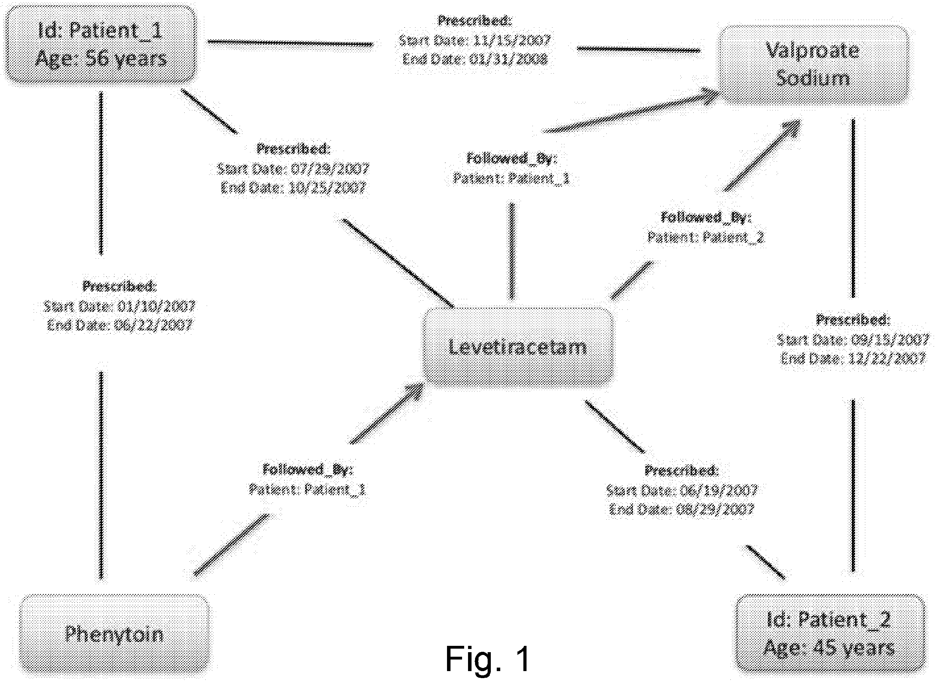

Fig. 1 shows an illustration of an exemplary directed graph representing prescription information for two different patients. The graph by default generates patterns consisting of monotherapies and can be customized to handle polytherapies. The generation of a treatment pattern from this graph is guided by the number of patients who are prescribed that particular pattern. For example, in the illustration shown in Fig. 1, patterns such as <*Phenytoin Levetiracetam*>, <*Levetiracetam Valproate Sodium*> and <*Phenytoin Levetiracetam Valproate Sodium*> exist and only the ones prescribed to a minimum threshold number of patients are analyzed for insight.

The present disclosure also provides a predictive model to predict whether or not a patient is likely to fail at least 3 subsequent AEDs and achieve refractory status at the time when the patient fails the first AED based on the medical history of patients and information gleaned from the treatment pathways. Patients identified by this model can be carefully monitored by physicians over time and can take specific drugs that may be more effective than standard ones, in an effort to prevent patients from achieving refractory status. The model may make use of an integrated healthcare dataset containing demographics, medications, diagnosis, procedures and encounters data for a large group of patients over a period of a plurality of years.

The model can be built using a predictive modeling pipeline comprised of constructing an appropriate cohort, followed by feature construction and selection. Finally, classification is performed using classifiers, which are evaluated with cross-validation supported by standard metrics such as C-statistic, precision and recall.

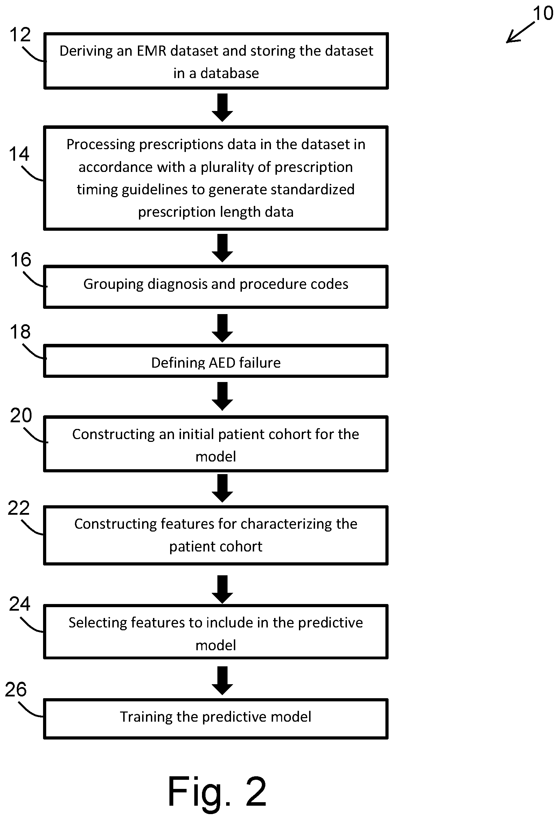

Fig. 2 schematically shows a flow chart of a method 10 of generating a predictive model in accordance with an embodiment of the present invention. Method 10 first includes a step 12 deriving an electronic medical record (EMR) dataset and storing the dataset in a database. In one preferred embodiment, the EMR dataset is derived from raw medical claims data including diagnosis, procedures and pharmacy claims spanning a period of time including one or more years and can be collected from different regions of a country. For example, the raw medical claims data can be data collected from different regions of the United States by IMS Health Surveillance Data Incorporated (SDI) medical database. In one preferred embodiment, the raw medical claims data incorporates patients from geographically dispersed regions along with third party and government payers.

Table 1 shows exemplary basic statistics for use in the EMR dataset calculated based on the raw data. The data consists of twenty-seven AEDs, four of which are rescue medications and are not treated as antiepileptic drugs. Table 2 shows the complete list of the 23 AEDs referenced in Table 1.

**Table 1**

| **Metric** | **Count** |
|---|---|
| Number of Patients | 20,596,917 |
| Number of Pharmacy Claims | 291,433,890 |
| Number of Diagnosis Claims | 1,206,477,159 |
| Number of Inpatient Claims | 2,790,966 |
| Number of Outpatient Claims | 8,608,737 |
| Number of ER Claims | 4,918,904 |
| Number of AEDs | 23 |

**Table 2**

| **Anti-Epileptic Drug** | | | |
|---|---|---|---|
| Carbamazepine | Divalproex Sodium | Ethosuximde | Ethotoin |
| Ezogabine | Felbamate | Fosphenytoin Sodium | Gabapentin |
| Lacosamide | Lamotrigine | Levetiracetam | Methsuximide |
| Oxcarbazepine | Phenobarbital | Phenytoin | Pregabalin |
| Primidone | Rufinamide | Tigabine HCl | Topiramate |
| Valproate Sodium | Vigabatrin | Zonisamide | |

| **Rescue Medications** | | | |
|---|---|---|---|
| Diazepam | Lorazepam | Clobazam | Clonazepam |

In order to ensure that the data being used for analytics is as accurate as possible, the EMR dataset is processed and standardized. For the present method, the dataset is modified and processed to remove inconsistencies and to suit the requirements of the model.

Along these lines, method 10 includes a step 14 of processing prescriptions data in the dataset in accordance with a plurality of prescription timing guidelines to generate standardized prescription length data. Step 14 eliminates certain clinically insignificant gaps between consecutive prescriptions of the same drug for each patient.

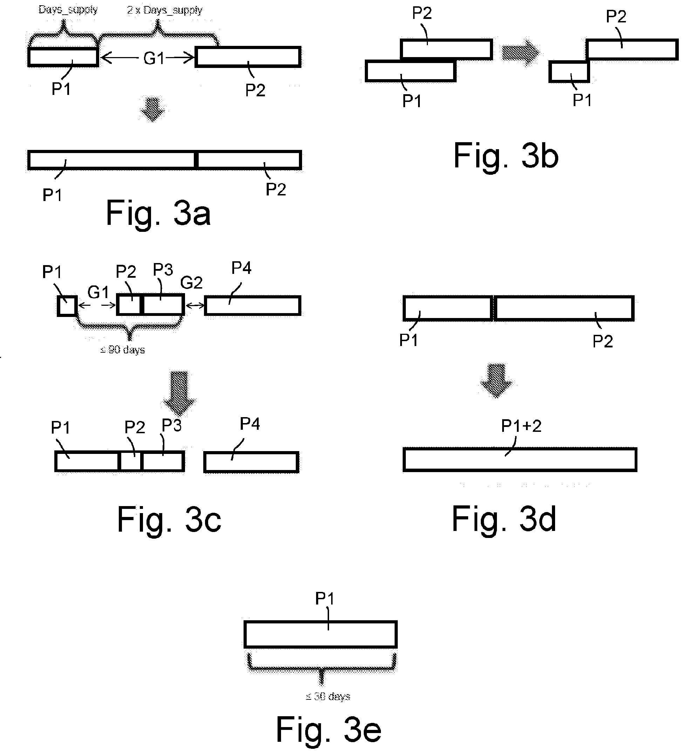

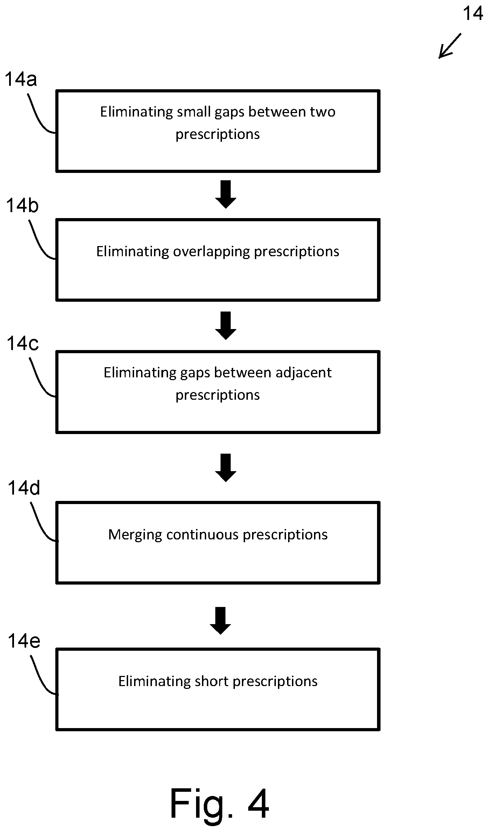

The substeps of step 14 are carried out in accordance with the sequence in of substeps 14a to 14e, as shown in Fig. 4. First, a substep 14a includes eliminating small gaps - i.e., gaps between two prescriptions of the same drug to a patient that are less than a predetermined threshold of time. As graphically illustrated in Fig. 3a, a small gap refers to a time gap G1 between two consecutive prescriptions P1, P2 of the same drug which is less than twice the time period, here a number days of supply of the earlier prescription P1. In this case, the earlier prescription P1 is extended to end on the beginning of service date of the later prescription P2.

Next, a substep 14b includes eliminating overlapping prescriptions, i.e., prescriptions whose time periods overlap with each other. As graphically illustrated in Fig. 3b, overlapping prescriptions are present for a patient when there are two consecutive prescriptions P1, P2 which overlap for certain time period, for example a number of days. The two prescriptions P1, P2 are merged for example by shortening one of the prescriptions P1, P2 so they do not overlap - here the earlier prescription P1 is shortened so that it ends on the beginning service date of the later prescription P2. Once the overlap is removed prescriptions P1, P2 become a continuous prescription which can be further processed as explained in substep 14d.

Next, a substep 14c includes eliminating gaps between adjacent prescriptions. As graphically illustrated in Fig. 3c, adjacent prescriptions are present for a patient when there are two consecutive gaps between prescriptions of the same drug within a predetermined time period of or less. In the embodiment shown in Fig. 3c, the predetermined time period is ninety days. In Fig. 3c, prescriptions P1, P2, P3, P4 are for the same drug and prescriptions P1 and P2 are separated by a time gap G1 and P3 and P4 are separated by a time gap G2. Accordingly, because gap G1 is less than ninety days, gap G1 is closed by extending prescription P1 to end on the beginning of service date of the prescription P2.

Next, a substep 14d includes merging continuous prescriptions. As graphically illustrated in Fig. 3d, continuous prescriptions are present when two consecutive prescriptions occur without a gap, i.e., the end date of the earlier prescription is the same as the start date of the later prescription. In Fig. 3d, prescriptions P1, P2 are merged to form a single prescription P1+2 beginning on the start date of prescription P1 and ending on the end date of the prescription P2.

Next, a substep 14e includes eliminating short prescriptions - i.e., prescriptions less than or equal to a predetermined threshold of time. As graphically illustrated in Fig. 3e, prescription P1 is eliminated because it is less than or equal to a predetermined threshold of time of thirty days. Although the above process is preferred, other parameters may be used based on the data analysis performed, for example, by clinicians.

Method 10 also includes a step 16 of grouping diagnosis and procedure codes. Most of raw healthcare datasets have diagnosis and medical procedures coded by standard systems of classification such as the International Classification of Diseases and Related Health Problems (ICD) and Current Procedural Terminology (CPT). Both CPT and ICD-9 codes help in communicating uniform information to the physicians and payers for administrative and financial purposes but for analytics these codes are grouped into clinically significant and broader codes presented by another scheme of classification named Clinical Classification Software (CCS) maintained by Healthcare Cost and Utilization Project (HCUP). The single level scheme consists of approximately 285 mutually exclusive diagnosis categories and 241 procedure categories. Step 16 includes mapping all the ICD-9 and CPT codes in the raw dataset to corresponding CCS codes for use in constructing appropriate features for the model. If CPT and ICD-9 codes do not have a corresponding CCS code, the CPT and ICD-9 codes are not processed in step 16 and are instead used in their raw form. Mapping files for converting CPT and ICD-9 codes into CCS codes are shown in Table 3.

**Table 3**

| Type | CCS code | ICD-9 Code |
|---|---|---|
| Diagnosis | Epilepsy Convulsions: 83 | 3450 |
| | | 34500 |
| | | 34501 |
| | | 3451 |
| | | 34510 |
| | | 34511 |
| | | 3452 |
| | | 3453 |
| | | 3454 |
| | | 34540 |
| | | 34541 |
| | | 3455 |
| | | 34550 |
| | | 34551 |
| | | 3456 |
| | | 34560 |
| | | 34561 |
| | | 3457 |
| | | 34570 |
| | | 34571 |
| | | 3458 |
| | | 34580 |
| | | 34581 |
| | | 3459 |
| | | 34590 |
| | | 34591 |
| | | 7803 |
| | | 78031 |
| | | 78032 |
| | | 78033 |
| | | 78039 |

| Procedure | CCS code | CPT code range |
|---|---|---|
| | Hemodialysis: 58 | 90918-90940 |

Method 10 further includes a step 18 of defining AED failure. In this embodiment, an AED treatment for a patient is said to have failed if the patient is prescribed another AED as a replacement of the current AED or as an addition to the ongoing treatment. For example, if the dataset indicates that a patient was prescribed Ezogabine from January 2013 to June 2013, and then was prescribed Pregabalin in replace of or in addition to Ezogabine in July 2013, Ezogabine is categorized as an AED failure for the patient.

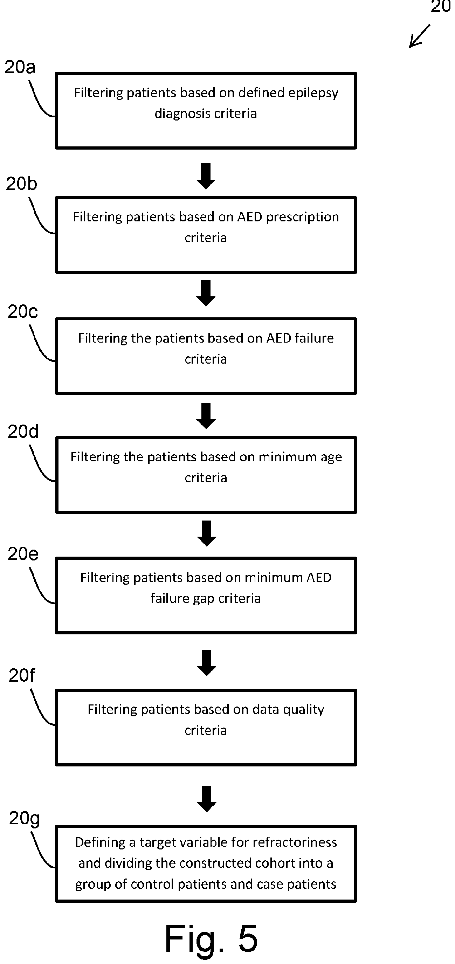

Method 10 further includes a step 20 of constructing an initial cohort for the model. Step 20 involves defining a sample of patients to be studied which meet some criteria relevant to the problem at hand. Criteria in step 20 are carefully designed by the domain experts, and an index date is set for every patient. The substeps of step 20 are shown in Fig. 5.

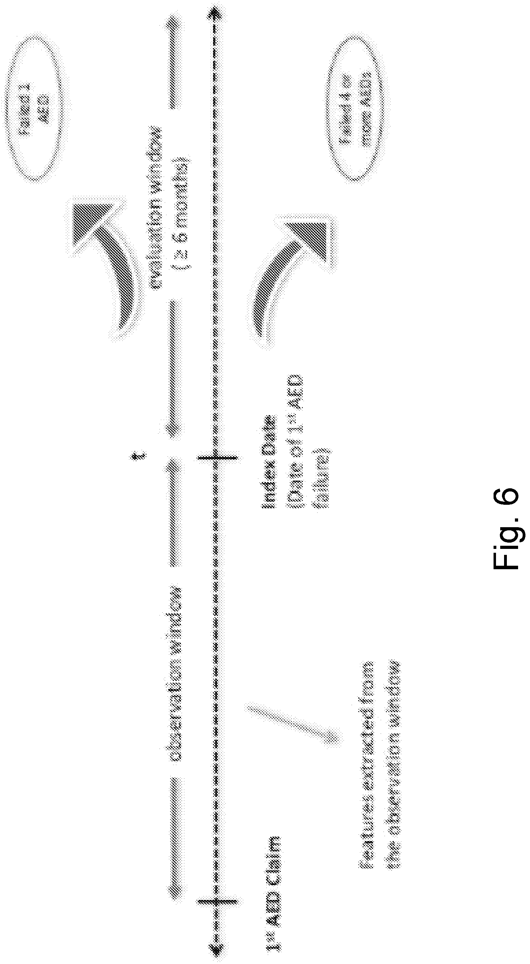

The index date defines a dividing point in the timeline of a patient, the period before which qualifies to be the observation period and the period after, becomes the evaluation period, as shown for example in Fig. 6. An object of step 20 is to find the patients within the dataset who have not benefited from the first AED prescribed to them and are likely to refract - i.e., have a statistical probability of refractoriness above a predetermined value. To accomplish this, the index date is set for a patient as the date of failure of the patient's first AED, and the patient's data before the index date is analyzed. For example, the entire population in the data set can consist of a set of adult epileptic patients with some additional inclusion and exclusion criteria carefully and extensively formulated by clinical experts.

Accordingly, as shown in Fig. 5, step 20 may include a substep 20a of filtering patients based on defined epilepsy diagnosis criteria to filter out non-epileptic patients. For example, to be included within the cohort, the patient must have at least one diagnosis claim of 345 (ICD-9 code for epilepsy diagnosis) or at least two claims of 780.39 (ICD-9 code for convulsions) at any time in the timeline of the patient. This criteria ensures the exclusion of all the patients which have not been diagnosed with any form of epilepsy and may have had one or less convulsions, thereby there is not substantial evidence to categorize the patient as an epileptic patient.

Step 20 may also include a substep 20b of filtering the patients based on AED prescription criteria. Patients not having at least one AED prescription at any time in the timeline are excluded. The AED prescription criteria throw out patients who are not as severe and were treatable with rescue medications.

Step 20 may also include a substep 20c of filtering the patients based on AED failure criteria. Patients not having at least one failure of an AED are excluded. In other words, for inclusion, a patient may be required to have at least two AED prescriptions which may or may not be distinct is excluded based on the above-mentioned definition of AED failure. The AED failure criteria is based on a time point at which refractoriness is predicted. In this embodiment, one AED failure is the minimum threshold because the prediction of refractoriness is at the time when the first AED has been tried and failed. In another embodiment, patients not having at least three failures of an AED are excluded. In a further embodiment, patients not having at least two failures of an AED are excluded.

Step 20 may also include a substep 20d of filtering the patients based on a minimum age criteria. Infants and teenagers in their early teens are excluded from the study by enforcing a minimum age criteria of for example sixteen years at the time of their first AED failure. Pediatric epilepsy patients are filtered out because pediatric epilepsy is treated differently from adult epilepsy and there could be certain types of seizures which only occur in children and not adults

Step 20 may also include a substep 20e of filtering patients based on minimum AED failure gap criteria. Patients who failed the first AED within 6 months of the prescription of the first AED are excluded to ensure that the AED was taken by the patient for a considerable amount of time before the AED failed. The minimum AED failure gap criteria provide sufficient time for the first AED to work before it fails.

Step 20 may also include a substep 20f of filtering patients based on data quality criteria. For example, the patient should have at least two consecutive years of minimum 75% eligibility in any of the pharmacy, diagnosis or hospital claims. The eligibility refers to the activity of a patient with respect to filing claims. More specifically, it is checked if a patient was active in filing a claim for either pharmacy or diagnosis or has hospital activity in a particular month. If a patient has activity in at least nine months out of the twelve months of a particular year and also has activity in at least nine months out of the twelve months of the following year, then the patient satisfies 75% eligibility for at least two consecutive years. The minimum eligibility criteria filters out patients who have long gaps between prescriptions or hospital visits. In addition to the minimum eligibility criteria, activity criteria may be used for each patient that requires the patient to have been active with respect to pharmacy claims in every quarter of each year. Data quality criteria makes sure patients who have no pharmacy claims for long periods of time are excluded because some patients may not comply with taking medications they are prescribed, which can add significant noise to the dataset.

Step 20 further includes a substep 20g of defining a target variable for refractoriness and dividing the constructed cohort into a group of control patients and case patients. For predicting patients with refractory epilepsy at an early stage it is helpful to discover factors contributing to refractory epilepsy. A patient can be effectively categorized as refractory by monitoring the seizure frequency over time; however, since seizures are not captured in the claims data, the number of AEDs tried on the patient is used as a proxy measure for refractory status in one preferred embodiment.

To maintain a clean distinction between refractory and non-refractory, in one preferred embodiment, refractory patients (i.e., case patients) are categorized as ones who have failed at least three distinct AEDs out of four, while non-refractory (i.e., control patients) are categorized as one who have failed exactly one AED - i.e., the patients each have exactly two distinct AED prescriptions. In another preferred embodiment, refractory patients (i.e., case patients) are categorized as ones who have failed at least two distinct AEDs out of four. The definitions of case patients and control patients are based on input by clinical experts. Some experts define patients who fail two AEDs as refractory which is why control patients are not defined as the ones having less than two AED failures. Patients are defined with four or more failures to be refractory so that extreme cases of refractory epilepsy can be defined using this model.

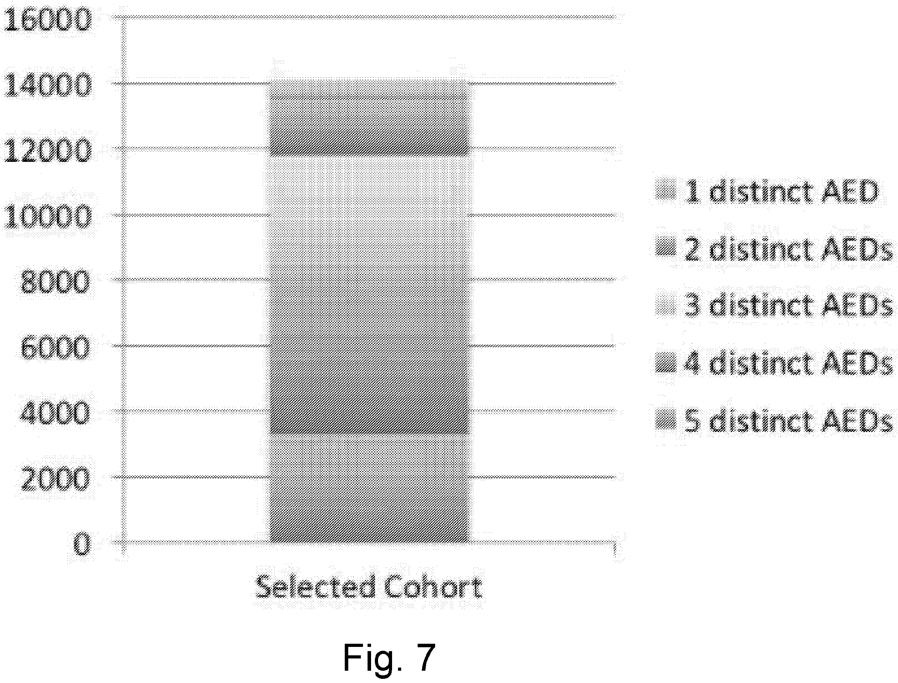

The raw data in the example, after being processed and funneled through the aforementioned multiple inclusion and exclusion criteria in step 20, results in 14,139 patients who have failed at least four AEDs and are potential candidates for being categorized as refractory based on input from domain experts. The example failure results from step 20, which are shown in Fig. 7, are then reviewed. Review of the results of step 20 indicates that within the AED distribution amongst the refractory candidate patients, there exist patients who have failed four or more AEDs but have repetitive AED prescriptions.

Method 10 further includes a step 22 of constructing features, including events, for characterizing the patient cohort. The claims data is used to extract diagnosis and procedural claims which are recorded as ICD9 and CPT code formats respectively in addition to the encounters and treatment information. All the information is represented as an event, e.g., prescription of a drug at particular time is an event. AED events are excluded since an aim is to predict if a patient is going to be refractory to AEDs. All the events are associated with a timestamp which reflect a temporal order in the dataset. If a patient has multiple events in a single visit, those events are grouped with the same timestamp. Demographic features are not temporal events, and are used as features more directly without temporal aggregation.

In an example, the initial set of features consist of 3,190 features extracted from the observation period of every patient excluding any information about the first AED prescribed. The observation period refers to the period before the index date all the way up to the first visit of the patient with the time period, irrespective of whether the first visit involved an epilepsy diagnosis. The method does not want overrule the possibility of other diagnoses/disease conditions influencing the refractory epilepsy status since epilepsy is associated with other comorbidities such as depression and hypertension. Accordingly, if a patient came into the hospital with a disease condition other than epilepsy, the hospital visit is still used to define the observation period. In one preferred embodiment, five different types of features are constructed - demographic features, comorbidity features, ecosystem and policy features, epilepsy status features and treatment features. Table 4 shows the summary of different exemplary features. The features are calculated in the 1 year period before the index date unless specified otherwise. In this example, the features are either Boolean or integers. The first column in the table shows the features categories selected in step 24. The second and the third columns show the feature description and the datatype of each feature followed by the last column showing the number of features generated to represent each feature mentioned. Some of the features used in the model are raw features used as is from the data set whereas some of the features are engineered to add clinical significance to the feature vector.

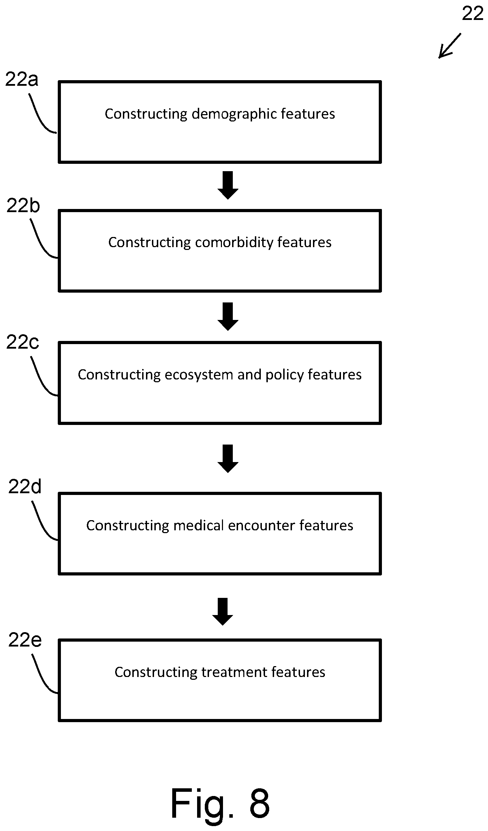

The substeps of step 22 are shown in Fig. 8, including a substep 22a of constructing demographic features from the dataset. The demographic features representing basic demographics of the patient such as age, gender and the geographic information of the patient. As shown in Table 4, the demographic features can include the first digit of the zip code of the patient, representing that the patient belongs to one of ten different geographic areas. The demographic features also include the age of the patient at the time when the patient failed his or her first AED, as categorized into three different bins namely "16 to 45 years", "45 to 65 years" and "greater than 65 years" and is used as a Boolean feature along with gender information.

Step 22 also includes a substep 22b of constructing comorbidity features from the dataset. The comorbidity features include features corresponding to the different comorbidities associated with epilepsy such as migraine, sleep related disorders, disorders and different kinds of mental disorders. The comorbidities may be specific, such as migraines, which are trivial to determine by looking for the appropriate diagnosis code in the data. By "trivial," it is meant that Migraine is associated with a single diagnosis code. All that is needed to determine if a patient was diagnosed with Migraine is to look for the appropriate diagnosis code. The comorbidities may also be generic, such as "Serious Mental Illness" which is determined by the presence or absence of mental illness related disorders such as psychosis and bipolar disorders, which in turn may have a range of diagnosis codes associated with them. The comorbidity feature set also involves comorbidity index scores such as the Charlson Comorbidity Index, as described for example in Charlson et al., "A New Method of Classifying Prognostic Comorbidity in Longitudinal Studies: Development and Validation." Journal of chronic diseases 40.5, pages 373-383 (1987), and Epilepsy Comorbidity Index, as described for example in St. Germaine-Smith et al., "Development of an Epilepsy-Specific Risk Adjustment Comorbidity Index," Epilepsia 52.12, p. 2161-2167 (2011), which are quantitative indications of the health of the patients. In the example shown in Table 4, the comorbidity features, except for the comorbidity index scores, are Boolean and represent the presence or absence of a particular comorbidity.

Step 22 also includes a substep 22c of constructing ecosystem and policy features from the dataset. The ecosystem and policy features include the factors which affect the care given to patients such as characteristics of the physicians treating the patients. The ecosystem and policy features also include insurance payer information because the type of payer represents the socioeconomic status of the patients, which in turn may affect the care provided to them. In the example shown in Table 4, the ecosystem and policy features are mostly boolean including information the prescribing physician's specialty and payer information.

Step 22 also includes a substep 22d of constructing medical encounter features from the dataset. The epilepsy status features are factors representative of the status of epilepsy of patients, including details about patient encounters. The patient encounter details may include type of visit, such as inpatient visit, outpatient visit or ER visit, and length of stay. Various checks for occurrence of seizures using diagnosis codes as proxies and monitoring of hospital and pharmacy activity of every patient are also included as epilepsy status features. In the example in Table 4, the epilepsy status include hospital encounter details.

Step 22 also includes a substep 22e of constructing treatment features from the dataset. The treatment features are features representative of to the treatment regimen and medical procedures undertaken by patients in the observation period. More specifically, a USP Classification Scheme can be used to group medications into categories based on therapeutic effects of the medications. In the example in Table 4, the treatment features include drug prescriptions. The medications have been grouped into higher level categories based on their therapeutic categories laid down by the U.S. Pharmacopeial Convention.

**Table 4**

| **Category** | **Feature Desc** | **Data Type of** | **No. of features** |
|---|---|---|---|
| **Demographics** | 1^{st} digit of ZIP code | Boolean | 10 |
| | Age at the time of first AED failure | Boolean | 3 |
| | Gender | Boolean | 1 |
| | **Total** | | **14** |
| **Comorbidity** | Affective disorder | Boolean | 1 |
| | ICD9 diagnosis code X in the period before the 1 year period before the index date | Boolean | 197 |
| | Neurological comorbidity | Boolean | 1 |
| | Substance abuse | Boolean | 1 |
| | Epilepsy comorbidity score | Integer | 1 |
| | Cardiovascular condition | Boolean | 1 |
| | Diagnosis CCS code X | Boolean | 283 |
| | Sleep disorder | Boolean | 1 |
| | ICD9 diagnosis code X | Boolean | 163 |
| | Porphyrin metabolism disorder | Boolean | 1 |
| | Osteoporosis | Boolean | 1 |
| | Autoimmune disorder | Boolean | 1 |
| | Charlson comorbidity | Boolean | 16 |
| | Obesity | Boolean | 1 |
| | Mental retardation | Boolean | 1 |
| | Liver condition | Boolean | 1 |
| | Diabetes | Boolean | 1 |
| | Diagnosis CCS code X in the period before the 1 year period before the index date | Boolean | 283 |
| | Renal insufficiency | Boolean | 1 |
| | Serious mental illness | Boolean | 1 |
| | Other mental disorder | Boolean | 1 |
| | Epilepsy related comorbidity | Boolean | 6 |
| | Charlson Comorbidity Index | Integer | 1 |
| | **Total** | | **965** |
| **Ecosystem & Policy** | Payer X | Boolean | 4 |
| | Physician prescribing the AED which failed is a general physician | Boolean | 1 |
| | Physician prescribing the AED which failed is a pain specialist | Boolean | 1 |
| | Physician prescribing the AED which failed has the word emergency in his/her specialty | Boolean | 1 |
| | Physician prescribing the AED which failed is a neuro specialist | Boolean | 1 |
| | Payer of first AED is X | Boolean | 4 |
| | **Total** | | **12** |
| **Epilepsy Status** | Medical procedure performed within 30 days before the index date | Boolean | 1 |
| | Occurrence of seizure based on icd9 code 345.X or 780.39 | Boolean | 1 |
| | Hospital encounter | Boolean | 1 |
| | CPT procedure code X | Boolean | 798 |
| | CPT procedure code X in the period before the 1 year period before the index date | Boolean | 765 |
| | Total length of stay in hospital | Integer | 1 |
| | Hospital encounter within 30 | Boolean | 1 |
| | Procedure CCS code X in the period before the 1 year period before the index date | Boolean | 240 |
| | Occurrence of seizure based on icd9 code 345.X only | Boolean | 1 |
| | Emergency room visit | Boolean | 1 |
| | CPT procedure code X | Boolean | 1 |
| | Procedure CCS code X | Boolean | 241 |
| | No of Months of pharmacy | Integer | 1 |
| | No of months of diagnosis | Integer | 1 |
| | No of months of hospital | Integer | 1 |
| | **Total** | | **2055** |

| **Treatment** | Treatment with medication class X within 30 days before the index date | Boolean | 3 |
|---|---|---|---|
| | Prescription of medication | Boolean | 140 |
| | **Total** | | **143** |

Method 10 further includes a step 24 of selecting features to include in a feature matrix for building and training the predictive model. Each patient is represented by a feature vector in the feature matrix. Table 5 shows an exemplary feature matrix including a few exemplary features for three patients.

**Table 5: Example Patient Vectors**

| PatientID | Age | Gender | Mental Illness | Depression | Any Convulsions in the last 1 year |
|---|---|---|---|---|---|
| P1 | 34 | F | 1 | 0 | 1 |
| P2 | 30 | M | 1 | 1 | 1 |
| P3 | 20 | M | 0 | 0 | 1 |

Step 24 includes performing a statistical test on the features to identify which of the features have a statistical significance value within a predetermined range. In one embodiment, the feature matrix, which is created before the feature selection step 24, consisting of both raw and engineered features is subjected to a feature selection process using ANOVA ("analysis of variable) F-value, which scores the features based on univariate F-test. Only a subset of the high scoring features - for example features within specified top percentile - found to be sufficient for prediction during parameter tuning to be used by the classifier in the predictive model are selected. In one preferred embodiment, the features having top 20% of overall ANOVA F-scores are selected.

The resulting sequential patterns from the sequential pattern mining can also be used as additional features in step 22 and can be selected for input into the predictive model in step 24.

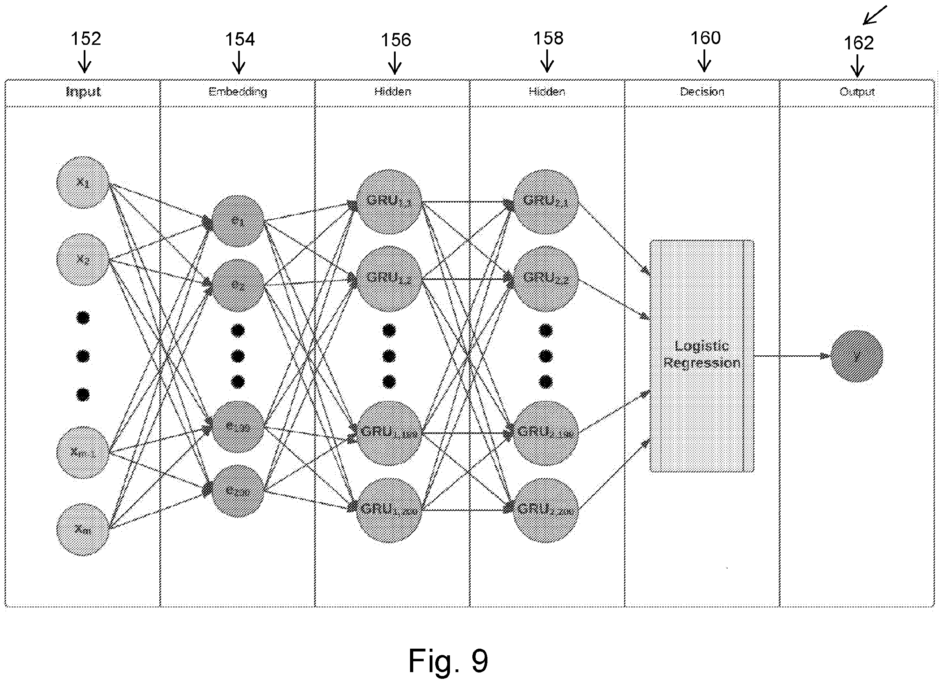

Method 10 further includes a step 26 of training the predictive model. In one preferred embodiment, the predictive model is a RNN 150 including the architecture shown in Fig. 9, including an input layer 152, an embedding layer 154, two hidden layers 156, 158 - recurrent layers with GRUs, a decision layer 160 including a logistic regression classifier and an output layer 162. For the input layer, for each patient, a sample is provided of size n from a univariate multilabel marked point process in the form of *(tᵢ, xᵢ*) for *i* = 1, ..., *n* . Each pair represents a set of grouped events. The multihot label vector *xi*∈ {0, 1}*^{p}* represents the medical events assigned at time *ti* , where *p* denotes the number of unique medical events. In other embodiments, in lieu of the RNN, a classifier may include algorithms in the form of a Linear Support Vector Machine (SVM) or a Random Forest classifier tuned appropriately for the purpose of training the predictive model.

For, if a patient has a diagnosis (Dx) claim with code 123 at t=0 and a Dx claim with code 345 and a prescription (Rx) claim with Drug3 at t=10, inputs for this patient will be a sequence of two vectors, since this patient has two visits at t=0 and t=10. Each vector is D-dimensional vector, where D is the number of possible medical code with value 1 at the corresponding index of the medical code in the vector and 0 otherwise. For example, a vector for the 1st visit would be [0 0 0 1 0 ....] where Diagnosis code 123 has index 4. The vector for the 2nd visit is [0 0 0 0 1 0 0 1 0 0 ....], where the diagnosis code 345 has index 5 and Drug3 has index 8. These two vectors together are the input for this patient. An output of the output layer is then a probability of refractoriness generated by logistic regression.

The predictive model is built to train on the patient data in the dataset before the index date and predicts whether the patient would eventually become refractory or remain non-refractory at the point when the patient experiences a first AED failure. In one preferred embodiment, the target variable is a binomial variable - i.e., refractory or non-refractory, and a Logistic Regression machine learning classifier is used for training the model in the decision layer of the RNN. In other embodiments, the RNN can include a decision layer in the form of a Linear Support Vector Machine (SVM) or a Random Forest classifier tuned appropriately for the purpose of training the predictive model.

A parameter to be tuned for linear SVM and Logistic Regression is the C-value, which specifies the regularization strength. Random forest on the other hand is an ensemble learning method for classification and operates by constructing a multitude of decision trees based on the training data and assigns the class that is the mode of the classes of the individual trees in the forest. The number of trees selected if optimally selected would increase the likelihood of obtaining accurate predictions. Another parameter for use in both the SVM and Logistic Regression classifiers is the class weight and use the 'balanced' value for the same. This parameter can be beneficial when the classes are highly imbalanced. For example, if a case to control ratio is 1:3, this parameter can help in penalizing the assignment of the majority class. In one embodiment, the C-value of SVM and Logistic Regression can be varied from 0.00001 to 1 and the number of trees for random forest can be varied from 150 to 300. Another parameter that can be varied is the number of features used as input to the model. The top percentile of features can be varied from 1 to 100 percent and for each percentile of features the classifier parameters are varied. The goal is to find the least number of features giving the best predictive performance using the most appropriate set of parameters.

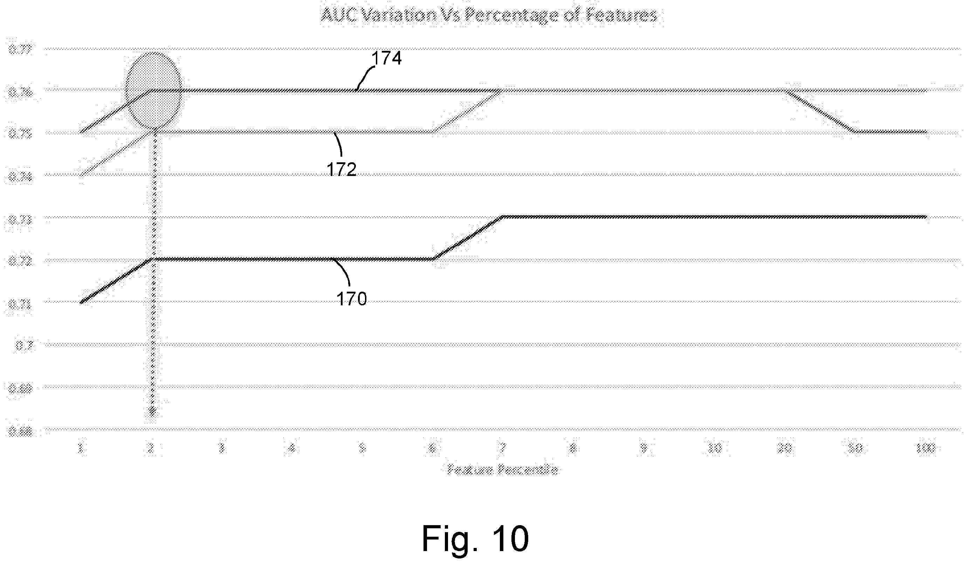

The distribution of AUC and Area Under the Precision Recall curve can be analyzed for one of more classifiers during the parameter tuning process for different percentiles of features. Fig. 10 shows an example of a graph with lines representing predictive models having three different classifiers - a first line 170 representing a predictive model with a SVM classifier, a second line 172 representing a predictive model with a Logistic Regression classifier and a third line 174 representing a predictive model with a RF classifier - on a graph of AUC versus the percentile of features included in the predictive model. With the SVM classifier, Fig. 10 shows an AUC of 0.73 using top 7% of the features while with Logistic Regression and Random Forest result in an AUC of 0.76 with the top 7% and 2% of the features respectively. Accordingly, the graph of Fig. 10 illustrates that the AUC does not improve on increasing the number of features used by the model, so the graph indicates that the maximum features to be included in the predictive model in such an example is the top 2%.

One aim is to learn an effective vector representation for the refractory or non-refractory status of patients at each timestamp *tᵢ*. The representation for the status of patients is used to predict future quantities about this patient regarding the possibility of becoming a refractory patient. To this end, RNNs are used to learn such patient representations. The state vector of RNNs, which is typically the last hidden layer, is treated as the latent representation for the patient status and is used for predicting refractory state of patients. The pretrained embedding layer includes Med2Vec or random initialization, and following the embedding layer, the RNN architecture include recurrent layers with GRUs are applied to extract features from sequential visit event data for each patient, in which the meaningful features are obtained automatically by the neural network by learning the weights of the features. The Med2Vec layer can be pretrained separately using multi-layer perception, which leverages only co-occurrence information. The Med2Vec layer is thus created to capture temporal dependency across events, e.g., visits, along with the co-occurrence information within each event. The output of the logistic regression classifier (decision layer) is used at the top of the output layer to make a prediction of the future refractory state of a patient.

Each layer of the RNN includes a plurality of RNN units. For example, a general hidden layer has many - 10s or 100s even 1000s - hidden units. Similarly, a RNN layer of the RNN (i.e., recurrent hidden layer) is composed by multiple RNN units (i.e., recurrent units) such as GRUs. The RNN units used can be simple RNN units as described in Le et al., "A Simple Way to Initialize Recurrent Networks of Rectified Linear Units," arXiv preprint arXiv: 1504. 00941 (2015)or more complex recurrent units such as Long ShortTerm Memory (LSTM) described in Hochreiter et al., "Long short-term memory, Neural Comput. 9, pages 1735-1780 (1997) and Graves et al., " A novel connectionist system for unconstrained handwriting recognition, I EEE Trans. Pattern Anal. Mach. Intell. 31, pages 855-868 (2009) or Gated Recurrent Units (GRU) described in Chung et al., "Empirical evaluation of gated recurrent neural networks on sequence modeling," arXiv preprint arXiv: 1412. 3555 (2014). Multiple units of RNNs can be stacked on top of each other to increase the representative power of the network. In one preferred embodiment, the RNNs are implemented with GRUs and the ADADELTA algorithm described in Zeiler, "ADADELTA: An Adaptive Learning Rate Method" arXiv [cs.LG] (2012) is an optimization algorithm used to train the network. It is a first order method and requires no manual tuning of a learning rate. The learning rate is dynamic and is computed on a per-dimension basis. Furthermore, the Dropout technique as described in Srivastava et al., "Dropout: a simple way to prevent neural networks from overfitting," J. Mach. Learn. Res. 15, pages 1929-1958 (2014)is used with a probability of 0.5 to prevent the networks from overfitting .

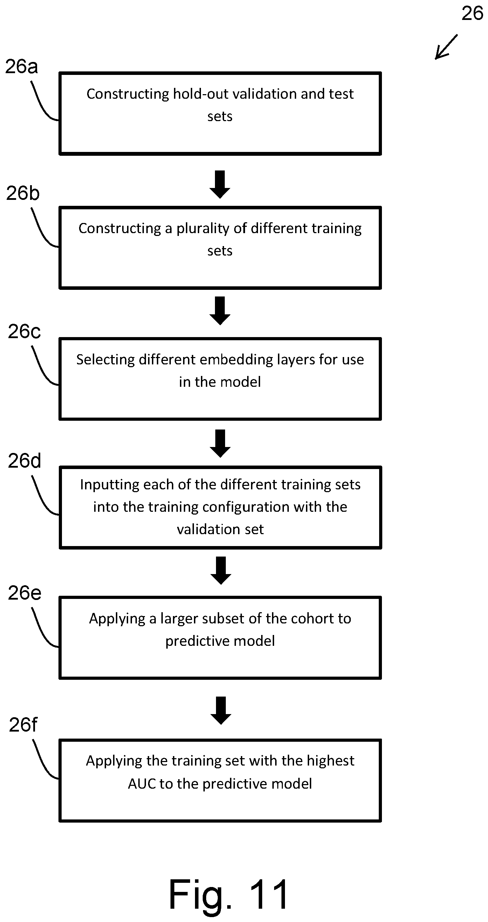

Step 26, as illustrated in Fig. 11, includes a substep 26a of constructing hold-out validation and test sets from the initial constructed cohort created in step 20. The validation set is a randomly sampled percentage of patients - in this example 30% - in the constructed cohort. The validation set is used repeatedly during the training process to evaluate current trained parameters. On the other hand, the test set, consisting of a percentage of patients - in this example approximately 20% - is mutually exclusive with the validation set and is used only after the training process is done to evaluate the performance of the best parameters verified by the validation set. Table 6 describes a brief statistics for the validation set and test set in this example.

**Table 6**

| **Metric** | **Validation Set** | **Test Set** |
|---|---|---|
| No. of Patients | 16,005 | 13,496 |
| No. of Case Patients | 1,810 | 1,455 |
| No. of Control Patients | 14,195 | 12,041 |
| No. of Distinct Medical Codes | 19,367 | 18,166 |
| No. of Diagnosis Codes | 7,741 | 7,381 |
| No. of Medication Codes | 1,604 | 1,501 |
| No. of Procedure Codes | 6,536 | 6,000 |
| No. of Drug-class Codes | 272 | 276 |
| Avg No. of Visits | 33.6 | 32.6 |
| Avg No. of Codes per Visit | 3.94 | 3.91 |
| Max No. of Codes per Visit | 121 | 84 |
| Avg Days between Visits | 21.1 | 21.8 |

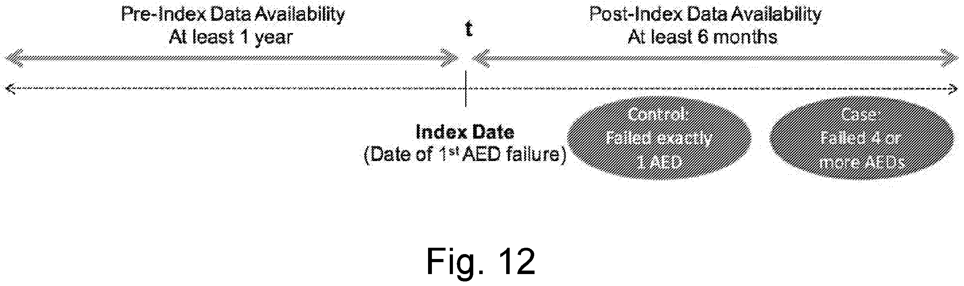

The construction of the validation and test sets can be filtered via Pre/Post-index data availability criteria, which dictates that in order for a particular patient to be included in the training set, the patient must have available data for at least one year before the index date and for at least six months after the index date as described in Fig. 12. That is, the first event of the patient should have occurred at least one year before the index date and the last event should have occurred at least six months after the index date. This criteria is crucial for defining both the validation set and the test set, as it allows for a clean definition of cohorts based on events immediately leading up to the index date.

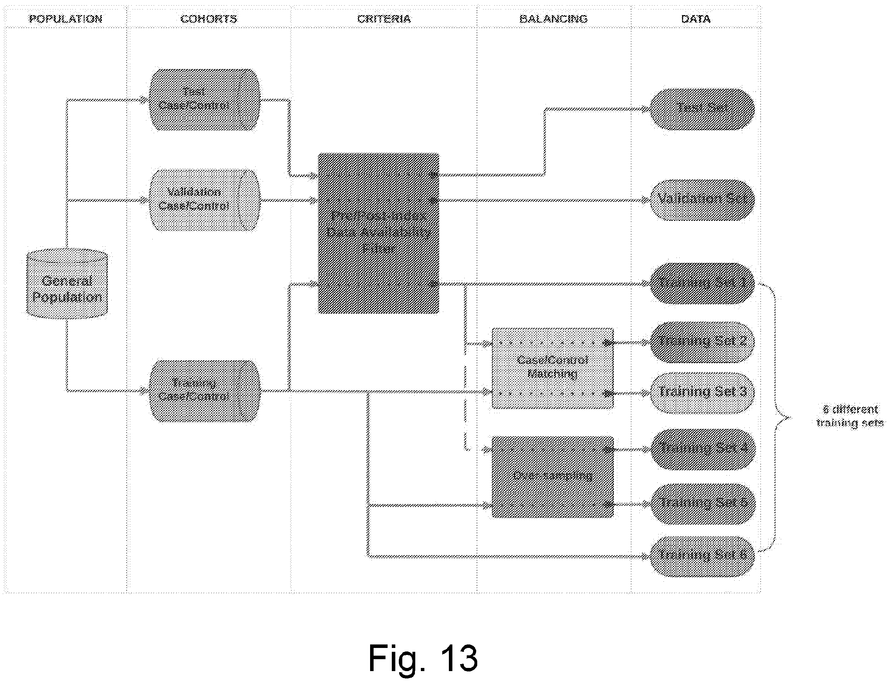

Referring back to Fig. 11, after substep 26a, step 26 further includes a substep 26b of constructing a plurality of different training sets. Fig. 13 shows the data processing pipeline for use in substeps 26a and 26b. If stronger constraints are introduced to qualify the patients in study, the number of available patients is reduced. On the other hand, it is axiomatic that the data would be noisy and contain outliers with looser constraint. Six different training sets are constructed and tested in the experiments in order to explore adequate balance between the size of data, especially the number of patients for training, and the quality of patient data. The different training sets may be defined by optional criteria restricting the patients in the set and/or cohort balancing strategies. In one preferred embodiment, the optional criteria include the Pre/Post-index data availability as described in substep 26a. The cohort balancing strategies can include Case/Control matching, over-sampling and unbalanced. For Case/Control matching, matching controls are identified by gender, zip code, and age within 5 years. Some case patients can be dropped from the cohort if there are no matched control patients. For over-sampling, multiple duplicated case patients are sampled with replacement - i.e., the same patient may be sampled multiple times - to get a similar number of patients with control patients. For unbalanced, a raw number of case and control patients are used without any balancing.

In this example, six sets of training sets are constructed. Set 1 is unbalanced dataset with pre/post-index data availability criteria (hereafter "pre/post-index criteria"). Set 2 and Set 3 are Case/Control matched set with and without pre/post-index criteria respectively. Set 4 and Set 5 are constructed with over-sampled case patients, with and without pre/post-index criteria respectively. Finally, Set 6 is unbalanced dataset without pre/post-index criteria, the natural and the biggest training set. The 1^{st} row, No. of Patients, refers to the number of original patients. The 2^{nd} and the 4^{th} row, No. of Case Patients and No. of Control Patients, represent the number of each group of patients AFTER criteria/balancing are applied.

**Table 7**

| **Metric** | **Set 1** | **Set 2** | **Set 3** | **Set 4** | **Set 5** | **Set 6** |
|---|---|---|---|---|---|---|
| No. of Patients | 37,398 | 8,298 | 15,826 | 37,398 | 85,684 | 85,684 |
| No. of Case Patients | 4,298 | 4.181 | 8.188 | 33,053 | 76,283 | 8,326 |
| No. of Distinct. Case Patients | 4,298 | 4,181 | 8,188 | 4,298 | 8,326 | 8,326 |
| No. of Control Patients | 33,100 | 4,117 | 7,638 | 33,100 | 77,358 | 77,358 |
| No. of Distinct. Medical Codes | 19,810 | 13,277 | 15,384 | 19,810 | 23,599 | 23,599 |
| No. of Diagnosis Codes | 9,640 | 6,439 | 7,052 | 9,640 | 11,347 | 11,347 |
| No. of Medication Codes. | 1,833 | 1,383 | 1,511 | 1,833 | 2,093 | 2,093 |
| No. of Procedure Codes | 8,048 | 5,193 | 6,102 | 8.048 | 9,851 | 9,851 |
| No. of Drug-class Codes | 286 | 259 | 266 | 286 | 305 | 305 |
| Avg No. of Visits | 33.5 | 32.1 | 28.2 | 33.5 | 32.6 | 32.6 |
| Avg No. of Codes per Visit | 4.3 | 4.3 | 4.3 | 4.3 | 4.3 | 4.3 |
| Max No. of Codes per Visit | 98 | 98 | 98 | 98 | 124 | 124 |
| Avg Days between Visits | 21.3 | 20.4 | 20.9 | 21.3 | 22.6 | 22.6 |

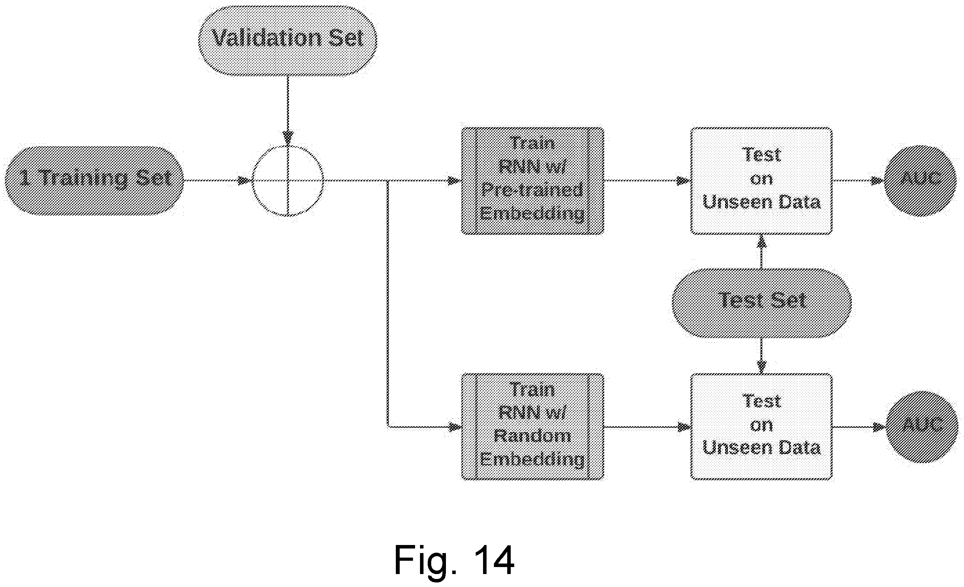

Step 26 also includes a substep 26c of selecting different embedding layers for use in the training configuration of the prediction. In the example shown in Fig. 14, one RNN includes a pre-trained embedding layer and another RNN includes a randomly initialized embedding layer. An embedding layer is a type of layer that usable in deep neural networks and used in Natural Language Processing (NLP) applications. An embedding layer is a kind of matrix and an input vector of the deep neural network, which is a one-hot or multi-hot vector in NLP in preferred embodiments, is multiplied by this matrix. One preferred embodiment uses either a matrix initialized with some random numbers or a matrix of which values are trained by other deep neural network. In one preferred embodiment, the pre-trained embedding layer is a Med2Vec embedding layer pre-trained using the Med2Vec technique, as described in E. Choi, A. Schuetz, W. F. Stewart, J. Sun, Medical Concept Representation Learning from Electronic Health Records and its Application on Heart Failure PredictionarXiv [cs.LG] (2016) (available at http://arxiv.org/abs/1602.03686), but further modified to fit the current architecture. Med2Vec is trained using our data. We choose dimensions for our dataset and number of hidden layers and units in each hidden layer. Hyperparameters like the number of hidden units can be selected through grid-search or Bayesian optimization.

The Med2Vec model is an advanced variation of the Word2Vec model that is based on the fact that the nature of medical data is similar with that of natural languages. For example, each single medical code acts as word in natural languages. In other embodiments, Word2Vec and GloVe models can be used to train the embedding layer, as for example described in Mikolov et al., Advances in Neural Information Processing Systems 26, Curran Associates, Inc., 2013, pp. 3111-3119 (Word2Vec) and Pennington et al., "Glove: Global Vectors for Word Representation," EMNLP (2014) (GloVe), respectively.

Med2Vec algorithm, which learns a layer to reduce the dimensionality of the input data down, e.g. a few hundred dimensions of clinically interpretable representations. To learn a layer, the Med2Vec algorithm calculates optimal feature weights to make a hidden layer from raw inputs. The dimension of input vector can be as great as, and the embedding layer maps the input vector to a selected lower dimension K, defining the number of columns in the matrix of embedding layer. The Med2Vec embedding layer has a N × K weight matrix W_{emb}, where N is the number of all possible medical codes, the dimension of raw input vector, and K is the dimension, the number of hidden units, of embedding layer. Table 8 shows the top ten input dimensions which have high weights between the input layer and the coordinate (hidden unit) 1 of the embedding layer. In other words, Table 8 shows that top 10 input dimensions among N input dimensions which have high weights value in the first column - i.e., coordinate 1 - of W_{emb}, embedding matrix (layer). The values W_{emb} of are trained via pre-training process and the training process of our architecture.

A clinical domain expert, for example a MD/PhD physician scientist, may perform a validation of all fully trained patient-level representation coordinates learned in the embedding layer with Med2Vec to verify the representation coordinates are meaningful.

**Table 8: Example of learnt representation by embedding layer. DIAG_^{∗} and PROC_* represent ICD9 diagnosis code and ICD9/CPT procedure code respectively.**

| **Medical Code** | **Decription** | **Annotation** |
|---|---|---|
| DIAG_34590 | UNSPEC EPILEPSY WITHOUT MENTION INTRACT EPILEPSY | Epilepsy, Convulsion |
| DIAG_34510 | GEN CONVUL EPILEPSY W/O MENTION INTRACT EPILEPSY | |
| DIAG_78039 | OTHER CONV ULSIONS | |
| DIAG_8208 | CLOSED FRACTURE UNSPECIFIED PART NECK FEMUR | |
| PROC_99202 | OFFICE OUTPATIENT NEW 20 MINUTES | |
| PROC-99308 | SBSQ HOSPITAL CARE/DAY 20 MINUTES | |
| DIAG_2948 | OTH PERSISTENT MENTAL D/O DUE CONDS CLASS ELSW | |
| DIAG_4019 | UNSPECIFIED ESSENTIAL HYPERTENSION | |
| PROC_99232 | SBSQ NURSING FACIL CARE/DAY MINOR COMPLJ 15MIN | |
| DIAG_V700 | ROUTINE GENERAL MEDICAL EXAM@HEALTH CARE FACL | |

In a step 26d, each of the different training sets are input into the training configuration with the validation set from substep 26a, producing a number of different results - twelve different results in this example. Table 9 shows the result AUCs (Area Under Curve) and we split those into 2 groups according to Pre/Post-index criterion for readability. In general, a better AUC is obtained without Pre/Post-index criteria, as in this case there are a greater number of training samples. Also, an unbalanced training set yields a higher AUC than does an artificially balanced set such as a case/control matched set or an oversampled set under the same other conditions. As a result, Training Set 6 which is unbalanced without Pre/Post-index criteria gives the best AUC of 0.7045 when a pre-trained Med2Vec embedding layer is used. An inference can be drawn that more training data without distorting the original distribution yields a better prediction performance.

**Table 9**

| **Balancing Method** | **With Pre/Post Index criterion** | | **Without Pre/Post-index criteria** | |
|---|---|---|---|---|
| | **Random Embedding** | **Med2Vec** | **Random Embedding** | **Med2Vec** |
| Case-Comol Matching | 0.6348 | 0.6502 | 0.6855 | 0.6796 |
| Unbalanced | 0.6679 | 0.6800 | 0.7028 | **0.7045** |
| Over-sampling | **0.6826** | 0.6684 | 0.7040 | 0.7025 |

A 'fine-tuning' approach is applied for training a deep neural network to benefit from even the patients not satisfying all the criteria from the substeps of Fig. 4 to be included in the study cohort. The entire architecture including RNN layers as well as the Med2Vec embedding layer is trained with a larger general cohort which has looser constraints first while the patients still need to have a same number of AED failures with our study cohort to be either case or control. Specifically, a larger subset of the cohort is applied to the RNNs in a substep 26e and then the training set from step 26c with the highest AUC is applied to the training set in a substep 26f. For example, the networks are trained with case and control patients from a population who satisfied diagnostic criterion of at least one 345. * or at least two 780.39 ICD9 code over their entire medical history. Then, the networks are trained again using Training Set 6.

The training set is used in step 26 to train the model with data prior to the index date all the way up to the first record of the patient (observation period). The rest of the data is used as a hold out set which is never used for training at any point in time including the feature selection phase.

Step 26 can also include cross-validation of the classifier. Cross-validation is a technique used to train a single specific classifier to see the performance variation according to the different values of the parameters of that classifier. Once the parameters for the classifier are decided through cross-validations, the classifier is evaluated on the hold-out (or called hold-off) set again. Cross-validation may be omitted for embodiments including deep neural networks since a training time for a deep neural network is much longer than traditional classifiers such as SVM (Support Vector Machine), RF (Random Forest) and LR (Logistic Regression). Instead, for deep neural networks, a separate `validation set' is used during the training process to check the performance of the parameters (weights in each layer for the case of neural networks). In one embodiment, ten-fold cross validation on the training set is used to tune the parameters and finally test the best model from cross validation on the hold out test set. The cross validation is considered as being ten-fold because ten 'partitions' of data are used for training and validation iteratively in leave-one-out way.

In step 26, the test set is used to objectively assess the predictive power of the trained model. The predictive power can be assessed based on various evaluation metrics such as area under the ROC curve (AUC), precision and recall. Table 10 shows the number of case and control patients in each of the two sets. The evaluation period for the predictive model begins exactly after the index date and extends up to the last record of the patient.

**Table 10**

| **Type of Dataset / Class** | **Case** | **Control** |
|---|---|---|
| Training | 28,485 | 81.984 |
| Hold cut Test | 5871 | 14.670 |

Table 11 below shows the twenty most predictive features in accordance with one embodiment of method 10, divided into three categories: indicators of level of activity within the medical system, indicators of comorbidities and indicators of epilepsy and epilepsy complexity.

**Table 11**

| **Indicators of level of activity within the medical system** |
|---|
| Number of claims prior to the past year with diagnoses classified as CCS 259: "residual codes; unclassified" |
| Number of months in the past year with claims containing any diagnostic code |
| Number of claims prior to the past year for diagnostic tests classified as CCS 227: "Other diagnostic procedures: interview; evaluation; consultation" |
| Number of claims in the past year for procedures classified as CCS 227: "Other diagnostic procedures: interview; evaluation; consultation" |
| Number of claims prior to the past year for radiology procedures classified as CCS 226: "Other diagnostic radiology and related techniques" |
| Number of claims prior to the past year for chemistry and hematology tests classified as CCS 233: "Chemistry and hematology" |
| Number of claims in the past month for any procedure |
| Number of claims in the past year for any procedure |
| Number of claims prior to the past year for therapeutic procedures classified as CCS 231: "Other therapeutic procedures" |
| Number of months in the past year with claims for any prescription |

| **Indicators of Comorbidities** |
|---|
| Age |
| Charlson Comorbidity Index score |
| Number of claims prior to the past year for diagnosis of essential hypertension classified as CCS 98: "Unspecified essential hypertension" |
| Number of claims at any past time for lipid-lowering drugs classified as USP Class: "Dyslipidemics HMG CoA Reductase Inhibitors" |
| Number of prescriptions at any time in the past for SSRIs or SNRIs |
| Number of claims at any past time for GABA agonists classified as USP Class: "Gamma-aminobutyric Acid (GABA) Augmenting Agents" |

| **Indicators of Epilepsy and Epilepsy Complexity** |
|---|
| Number of claims prior to the past year with an epilepsy diagnostic code classified as CCS 83: "Epilepsy; convulsions" |
| Number of claims in the past year with an epilepsy diagnostic code classified as CCS 83: "Epilepsy; convulsions |
| Epilepsy Comorbidity Score |
| First antiepileptic drug is prescribed by a neurologist (yes/no [1/0]) |

Steps 12 to 26 and the sequential pattern mining can be analyzed to generate insights with respect to treatment pathways for antiepileptic drugs across various age groups, providers, and type of epilepsy; and steps 24 and 26 can be reiterated to tweak the predictive model and further tune the parameters.

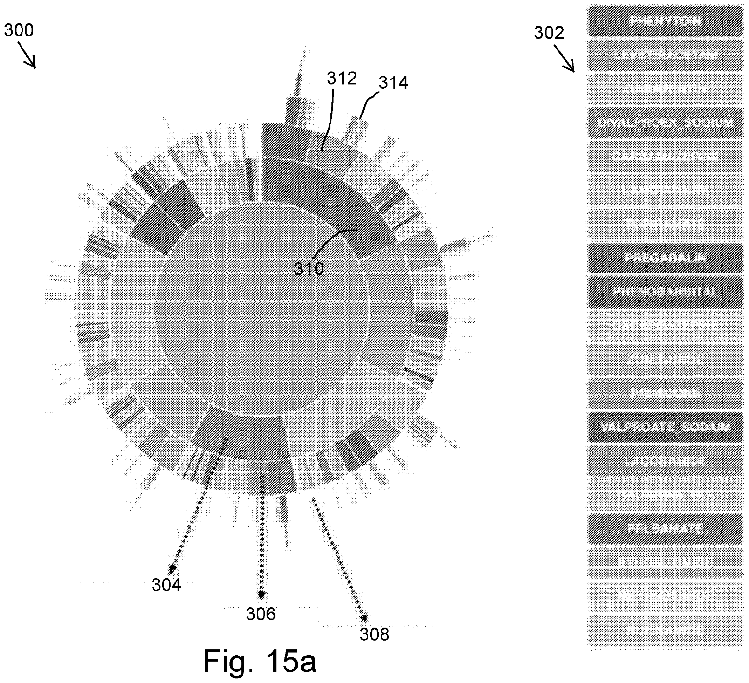

The analysis can include selecting only those patients which have been conclusively diagnosed with epilepsy based on the epilepsy diagnosis criteria mentioned in substep 20a and are at least sixteen years of age at the time of their first visit. The analysis can include generating a sunblast visualization 300, as shown in Fig. 15a, of the frequent treatment pathways is based on an extensive analysis of an exemplary data set including 3,949,404 patients satisfying the aforementioned diagnosis and age criteria. The drugs in the sunblast visualization 300 are color coded as shown in a key 302. Visualization 300 includes a first concentric circle 304 illustrating drugs that constitute the first line of treatment. A second concentric circle 306 illustrates a second line of treatment that follows the first line of treatment and a third concentric circle 308 illustrates a third line of treatment that follows a second line of treatment. First concentric circle 304 includes a plurality of arcs, with each arc representing a different one of the drugs shown in key 302. The arcs in the first concentric circle each have a length representation of a number of prescriptions in the first line of treatment from the data set. For example, an arc 310 in the first concentric circle 304 represents the most commonly prescribed first treatment drug - Phenytoin - and is longer than the other arcs in first concentric circle 304. The drugs that follow a first line of treatment of Phenytoin in the data set in a second line of treatment are shown in directly radially outside of arc 310. For example, an arc 312 in second concentric circle 306 has a length that represents the number of the patients of the data set that were prescribed Phenytoin first, and then were next prescribed the drug Levetiracetam. The drugs that follow a first line of treatment of Phenytoin and a second line of treatment of Levetiracetam are shown in a third line of treatment directly radially outside of arc 312. For example, an arc 314 in third concentric circle 308 has a length that represents the number of the patients of the data set that were prescribed Phenytoin first, and then were next prescribed the drug Levetiracetam, then were prescribed the drug Gabapentin as a third treatment.

By analyzing the first line of treatment, there does not seem to exist any one particular drug which distinctly stands out and can be categorized as the treatment of choice irrespective of the patient's age and type of epilepsy, which corroborates the fact that there is no universally accepted standard of care for epilepsy. However the top 3 most frequently used first line of care consists of AEDs Phenytoin, Levetiracetam and Gabapentin. The 2nd line of treatment is extremely variable and consists of multiple different AED choices but it is observed that the popular first line drugs are usually prescribed in repetition for most of the patients.

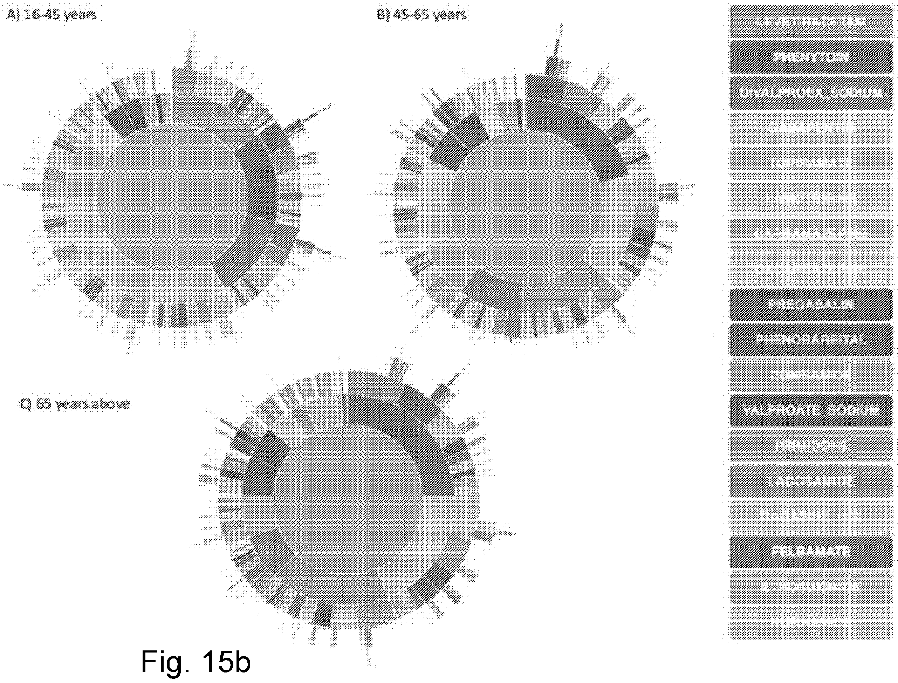

As shown in Fig. 15b, sunblast visualizations can also be generated for different age groups. Adult epileptic patients in the data set can be divided into three different age groups: (1) 16 to 45 years, (2) 45 to 65 years, and (3) 65 years and above. There are times when AEDs that work well as the 1st line of treatment for young adults may not be the best choice for the older epileptic population. Fig. 15b shows the treatment pathways for the aforementioned age groups. The visualizations in Fig. 15b suggest that Levetiracetam is the most popular choice amongst the AEDs as the first line of treatment for patients in the age group of 16 to 45 years. With higher age groups clinicians prefer to begin treatment with Phenytoin, whereas Gabapentin is the second most popular choice as the first drug followed by Levetiracetam. Lamotrigine and Topiramate are also used as the first line drug for younger patients in the age group of 16 to 45, but is not preferred for patients above 45 years of age. For the second line of treatment, there exists a lot of variability in the choice of AED for patients in the 16 to 45 age group whereas for patients more than 45 years of age Levetiracetam, Gabapentin and Phenytoin are equally common. A common phenomena observed in all the three age groups is that the first line drug is usually repeated after a gap of at least 90 days.

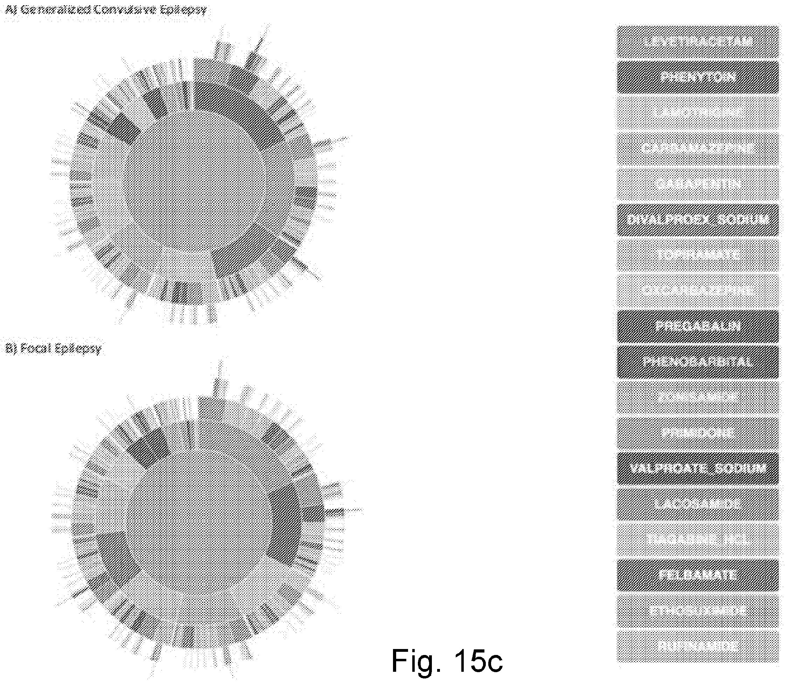

As shown in Fig. 15c, sunblast visualizations can also be generated for different types of epilepsy. The type of epilepsy diagnosed for patients can also be an influential factor in determining the treatment plans for patients. Clinicians identify two main types of epilepsies namely Idiopathic Generalized Epilepsy (IGE) which is diagnosed when patients experience electrical impulses throughout the entire brain and Symptomatic Localization Related Epilepsy (SLRE) epilepsy which involves seizures affecting only one hemisphere of the brain. The present disclosure categorize the patients into two cohorts based on the type of epilepsy diagnosed based on the first occurrence of the corresponding ICD9 diagnosis code. Fig. 15c shows sunblast visualizations of the treatment pathways for the two types of epilepsies. For patients diagnosed with IGE the clinicians prefer to recommend Valproic acid over Lamotrigine or Topiramate. In Fig. 15c, it is observed that Divalproex Sodium which is a derivative of Valproic acid is amongst the top three AED choices for the first line of treatment preceded by popular choices Phenytoin and Levetiracetam. Lamotrigine and Topiramate are also prescribed as the first line of treatment to 9% and 8% of the patients which corroborates the expert recommendations. The second line of treatment is case of IGE patients, is dependent on the AED prescribed as part of the first line of treatment. From the data it can be observed that the best choice of AEDs after prescription of Divalproex Sodium are Lamotrigine and Levetiracetam. Levetiracetam also seems to be the popular choice of treatment for patients who are prescribed Lamotrigine as the first drug.

In the case of SLRE, the clinicians prefer Carbamazepine, Gabapentin, Levetiracetam, Oxcarbazepine, Phenytoin, Topiramate and Valproic Acid when deciding the first line of treatment. In Fig. 15c, the visualization for SLRE shows the use of the aforementioned medications as the preferred choices for the first line of treatment although a lot of variation in the second line of treatment. It has been observed that Levetiracetam which is the most popular choice as the first prescribed AED in case of SLRE is followed primarily by Lamotrigine, whereas Phenytoin, Gabapentin and Divalproex Sodium are all followed primarily followed by Levetiracetam which is in alignment with recommendation from experts as well.

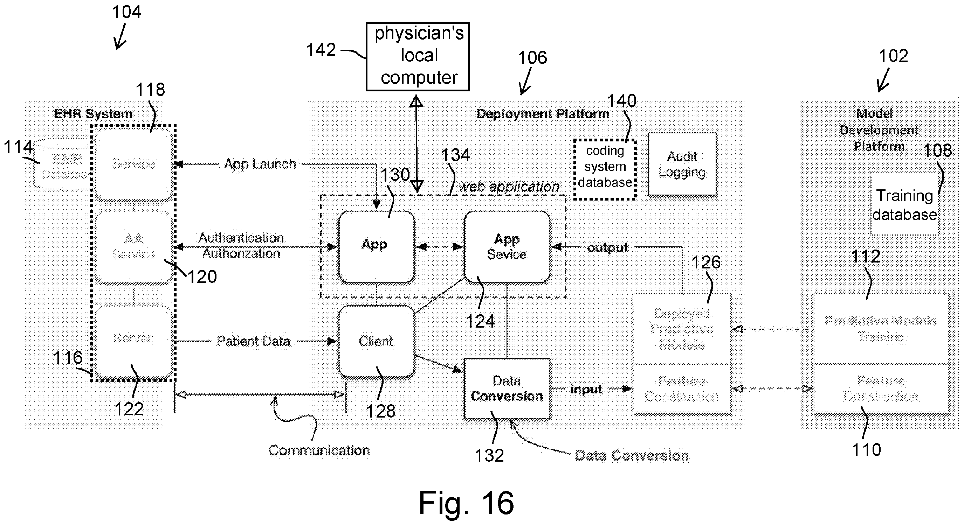

Fig. 16 illustrates a computer network 100 in accordance with an embodiment of the present invention for deploying the predictive models. Network 100 includes a development computer platform 102 configured for developing the predictive models as described above with respect to the method of Fig. 2, an EMR system 104 configured for providing electronic health record data and a deployment computer platform 106 configured for receiving inputs, running inputs through the predictive models and graphically displaying an output of the predictive models to a user.

Development computer platform 102 includes a training database 108 including the EMR data described with respect to method of Fig. 2, a feature construction tool 110 configured for carrying out some or all of the substeps of step 22 and a predictive model training tool 112 configured for carrying out some or all of step 26.

EMR system 104 includes a medical record database 114 and deployment tools 116 including an interoperability application program interface tool 118, an authentication and authorization server 120 and a data interface server 122. EMR database 114 stores the EMRs of patients serviced by a healthcare group, which can be an integrated managed care consortium or integrated health care system, operating facilities with access to EMR system 104. EMR database 114 includes health care data transaction and contents that can be translated to resources by deployment tools 116 for interoperability support. In the interoperable networks, the data is formatted in a specification to capture and store health data into forms known as resources. The resources can define generic templates for each type of clinical information, including prescriptions, referrals, allergies, and instances of these resources can be created to contain patient related information. The resources, in general, contain small amounts of highly specific information and therefore are linked together through references to create a full clinical record for each patient. Multiple linked resources are then brought come together to construct an EMR system in EMR database 114. More specifically, the resources can be Fast Healthcare Interoperability Resources (FHIR) developed by Health Level Seven International (HL7). Each resource shares the following in common: (1) a URL that identifies it, (2) common metadata, (3) a human-readable XHTML summary, (4) a set of defined common data elements, and (5) an extensibility framework to support variation in healthcare.

Interoperability application program interface tool 118 provides a platform for external applications. Authentication and authorization server 120 provides a security layer for interacting with external applications. Data interface server 122 provides a standardized format for the exchange of data. In one preferred embodiment, deployment tools 116 are in the form of a SMART on FHIR system, with tool 118 being in the form of a Substitutable Medical Applications and Reusable Technologies (SMART) platform, server 120 being in the form of an OAuth 2.0 compliant server and server 122 being in the form of a Fast Health Interoperability Resources (FHIR) server.

Deployment computer platform 106 includes a refractoriness prediction application service 124 for receiving a user request to run deployed predictive models provided to application service 124 by a predictive model deployment tool 126 and, in response, coordinating the running of the deployed predictive models. Predictive model deployment tool 126 can be provided with a feature construction module and a predictive modeling module. The deployed predictive models are the completed predictive models trained by tool 112 of development computer platform 102. Deployment computer platform 106 further includes a client 128 for interacting with server 122 and an epilepsy refractoriness prediction application 130 configured to interact with a medical practitioner, for example a physician seeing a patient, via a graphical user interface (GUI) and displaying a predictive output on the GUI. In embodiments where deployment tools 116 are in the form of a SMART on FHIR system, client 128 is a FHIR client and application 130 is a SMART enabled application. Client 128, in response to inputs from the practitioner received via prediction application 130, receives EMR data for the patient being seen by the practitioner from database 114 via data interface server 122. Prediction application 130 can be configured to handle both EMR and claims, as both use the same coding schemes. The patient data is provided by client 128 to application service 124 and a data conversion tool in the form of an epilepsy feature mapping tool 132 formatted as dictated by feature construction tool 110. Application service 124 is a backend service that coordinates operations between a prediction request entered by the practitioner and execution of predictive models. Prediction application 130 responds to the launch of deployment computer platform 106 on the physician's local computer, interacts with authorization and authentication server 120 to obtain authorization for accessing the EMR data in EMR database 114 and initiates transactions with data interface server 122. Prediction application 130 also maintains the state of the transactions at data interface server 122 and execution of predictive models, shows the state to the users on the physician's local computer browser, and provides an output representing an epilepsy refractoriness prediction from the predictive model on the GUI.

SMART on FHIR authorization supports both public and confidential app profiles. In one embodiment, a confidential app profile is used for deploying deployment computer platform 106 to increase security assurance. Before prediction application 130 can run against the EMR database 114, prediction application 130 is registered with the EMR's authorization service provided by the authorization and authentication server 120. In one embodiment, prediction application 130 is registered as an OAuth 2.0 client in authentication and authorization server 120.

Deployment computer platform 106 extracts relevant data in order to run the predictive models and produce results with the flexibility to work on any given system operating is accordance with the specifications an API, for example the specifications of FHIR. Once deployment computer platform 106, more specifically client 128, procures relevant data from the patient's EMR in database 114, the data is converted to the feature set by and used as an input to the predictive models exported from platform 102. After the predictive models finish executing with the input feature set, the results are visualized to the user on the GUI.

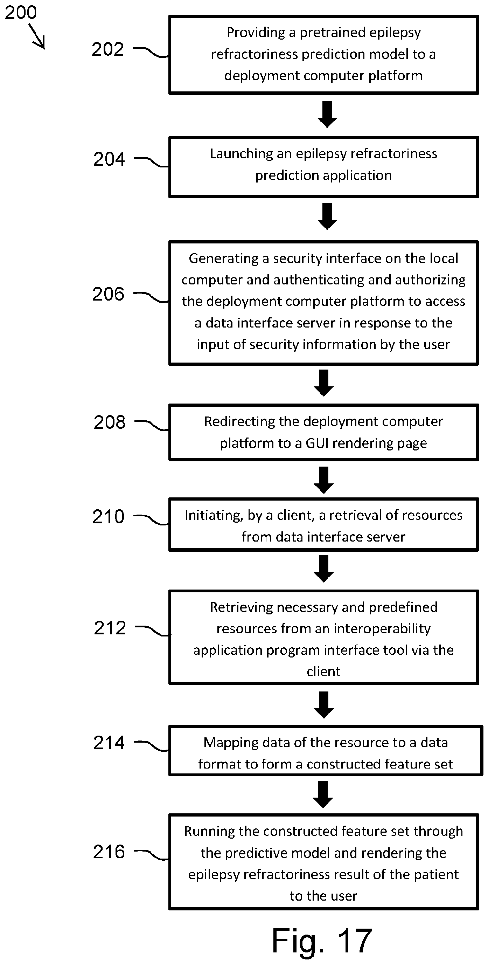

Application 130 and application service 124 together form an epilepsy refractoriness prediction generator 134, which in some preferred embodiments is a web application, for generating User interface and user experience components, e.g., the GUI. User interface and user experience components can be implemented in either application 130 or application service 124, depending on the development technology. Application 130 is configured to properly redirect the launch request to the viewer page in order for the status and result to be displayed in the EMR context. The user interface and user experience display can include three stages, as described further with respect to Fig. 17. First, there is a security stage to obtain authorization. A second stage involves getting data to be used as an input to the prediction models. A third stage involves executing the models and displaying the result on the GUI. In one embodiment, the first stage involves using OAuth 2.0 for security, the second stage involves using Web Socket, which allows browser to communicate with an app server, to show the status of transactions and the third stage involves using a programming language such as JavaScript to reload the outcome of predictive models on the results page.

In some preferred embodiments, where generator 134 is a web application, generator 134 contains both back-end and front-end capability, with back-end service modules of generator 134 being configured for working with a library of client 128. The back-end service modules can manage and control the entire work flow of web transactions within deployment computer platform 106. The back-end service modules can work with front-pages, such as for example SMART on FHIR's launching page, redirect page, in-progress page, and output pages.

Deployment platform 106 can also be provided with a coding system database 140, which can be embedded into deployment platform 106 or provided as a service from external entity. Either way, a query for the coding translation can be implemented in application service 124. The coding system database 140 is used to support interoperability in health information exchange between clinical systems that use different coding systems. To provide consistent contents for input signal to the predictive models, coding system database 140 allows health data elements received from EMR system 104 to be converted to a matching coding system, i.e., a coding system used to communicate with the predictive models in deployment platform 106. Database 140 can contain well-known coding system definitions and translation tables for each coding system.

The data conversion by feature mapping tool 132 can be critical in dictating the output quality of deployment platform 106. EMR data retrieved from EMR system 104 by client 128 is converted by tool 132 to an input format that predictive models can understand when they are executed. The data conversion is highly dependent on the model development, and the logic used for predictive model development is shared by platform 102 with platform 106. Any changes made during model development related to the feature construction are used to modify tool 132 so that better quality input signal can be generated.

Accordingly, although development computer platform 102 is not directly involved in the real time predictions provided by deployment computer platform 106, the feature mapping in the deployment platform 106 highly depends on the feature construction used in the modeling process. The feature construction methods from the method of Fig. 2 are provided to feature mapping tool 132 so that an implementable matrix for the feature mapping can be developed for mapping the patient data for use by application service 124. In preferred embodiments, the features include demographic features, comorbidity features, ecosystem and policy features, medical encounter features and treatment features. In one embodiment, feature mapping tool 132 reformats features in the EMR data from database 114 and represents at least some of the features in the data as events, as described above in step 22 of method 10. All the events are associated with a timestamp which reflect a temporal order in the dataset. If a patient has multiple events in a single visit, those events are grouped with the same timestamp. Feature mapping tool 132 also creates a feature set including those features selected in feature selection step 24 of method 10, such that the feature set input into the predictive models include features that are most statistically predictive of refractoriness.

For example, the EMR data from the resources can be provided by client 128 to conversion tool 132 and data elements of the EMR data can be mapped into a data model identifier. In one exemplary embodiment, data elements of FHIR data are converted to OMOP Concept ID as defined by Spaceship. If FHIR data elements are not mappable, those data elements are excluded in the data set (i.e., event data) input into the deployed predictive model. The event data can have a format in which the prefix indicates the type of data elements. For the FHIR data elements, for example, medical conditions are mapped from ICD-9 or ICD-10 codes, medical procedures are mapped from CPT code and the drugs prescribed can be mapped from the NDC's general name with all spaces replaced with "_". The mapped data elements are then passed through the feature construction and predictive model of tool 126.

Data conversion is a 1:1 module with the development platform and deployment platform 106. Therefore, deployment platform 106 needs to maintain separate data conversion for each different development platform. In others word, if a new development platform, for example based on a different programming language, is used for the developing the predictive model, a tool 132 needs to be redeveloped for the new development platform.

In creating feature mapping tool 132, human intervention can be employed to extract the implementable matrix from the feature construction due to the complexity of feature construction. Guidelines can be provided to the model developers for this purpose. The accuracy and completeness in which the patient data can be mapped to feature set affects the quality of predictive outcome.

In preferred embodiments, deployment platform 106 is designed to be launched from EMR systems. However, a stand-alone launch can also be developed (for mobile apps) with different SMART on FHIR scope parameters.

Fig. 17 shows a flow chart illustrating a computerized method 200 of generating and outputting of epilepsy refractoriness predictions in response to inputs of patient EMR data. Method 200 includes a step 202 of providing deployment platform 106 with a predictive model trained in accordance the method of Fig. 2. The predictive model may be trained solely with the data in training database 108, or periodically retrained using the real-time EMR data present in EMR database 114. In some embodiments where the predictive model is periodically retrained in accordance with step 26 of method 10, for example every 1 to 12 months, the EMR data from database 114 can be processed in accordance with steps 12 to 16, 18. In other embodiments where the predictive model is periodically retrained, feature selection step 24 can be repeated to ensure that the features most relevant to refractoriness prediction are selected for inclusion in the predictive model.

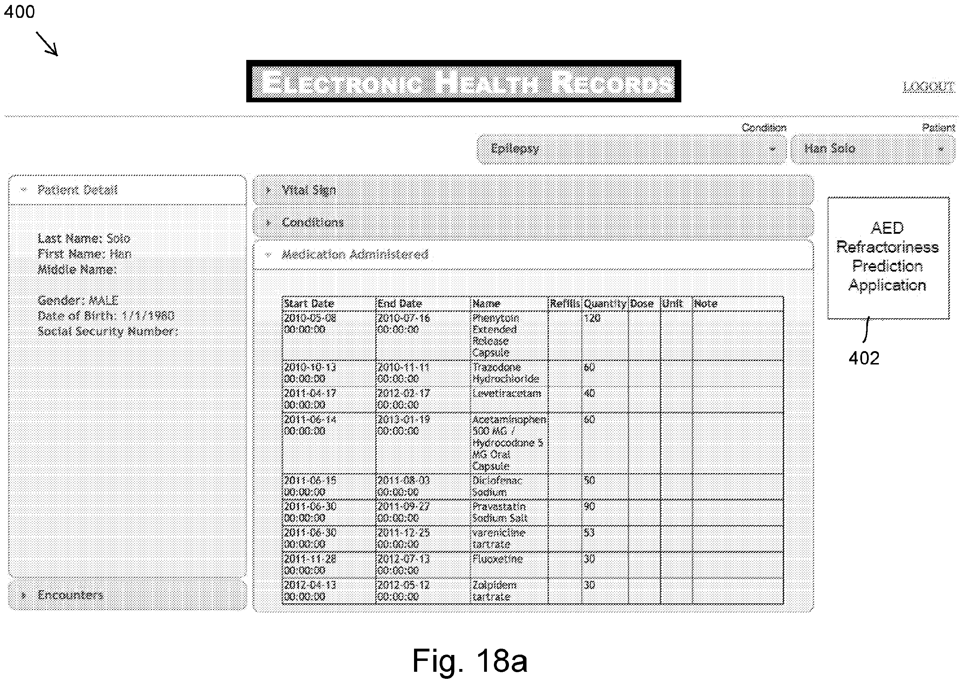

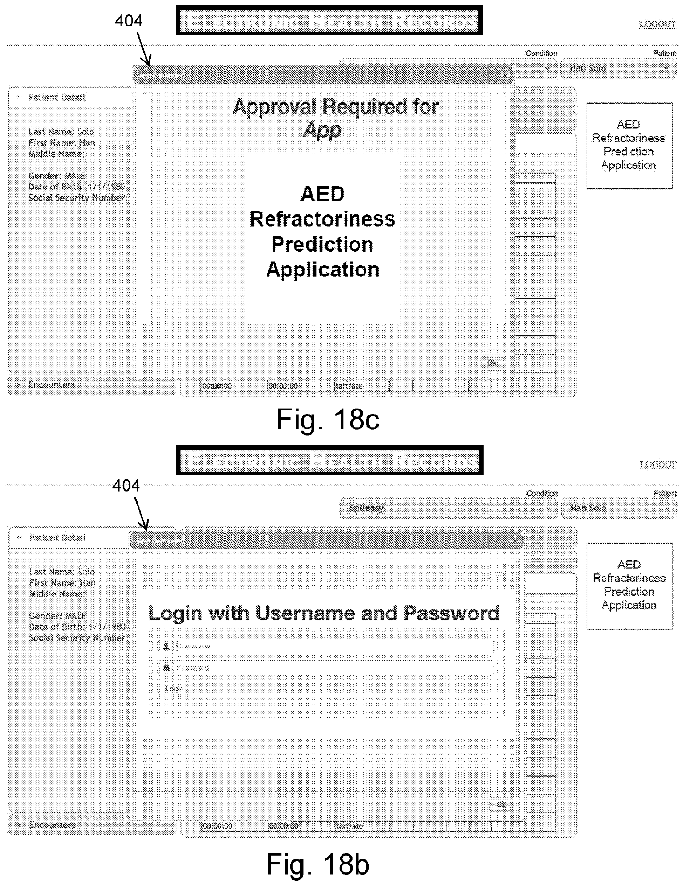

Method 200 also includes a step 204 of launching application 130 in response to initiation of application 130 in interface tool 118. Interface tool 118 displays a GUI 400, which is shown for example in Fig. 18a, on a physician's local computer for example in the physician's office that includes an icon 402 representing application 130. As shown in Fig. 18a, the icon 402 is accessible after the patient's EMR has been opened, such that the physician's local computer has already received the patient's EMR from EMR database, allowing application 130, once activated and (authenticated and authorized as discussed in step 206), to immediately access the patient's EMR. Upon selection of the icon by the user, i.e., practitioner, interface tool 118 launches application 130 on the physician's local computer. Authentication and authorization server 120 then, in a step 206 of method 200, generates a security interface 404 on the physician's local computer, as shown in Figs. 18b and 18c, and authenticates and authorizes deployment computer platform 106 to access data interface server 122 in response to the input of security information by the practitioner. In some embodiments, authentication and authorization server 120 requests access tokens from authentication and authorization server 120. Once deployment computer platform 106 is authorized and authenticated, method 200 proceeds to a step 208, in which deployment computer platform 106 is redirected to a GUI rendering page on the physician's local computer.

In a next step 210, while application 130 maintains the state of the transaction with interoperability application program interface tool 118, client 128 initiates retrieving resources from data interface server 122. Then, in a step 212, using the authorized state, necessary and predefined resources are retrieved from data interface server 122 via client 128. Each time a data is retrieved from EMR database 114 and converted into a resource, the data of the resource - i.e., the patient's EMR - is mapped in a step 214 via epilepsy feature mapping tool 132 to the data format of the feature set selected in the predictive model of method 10. The status of the mapping is reported to the user via a GUI rendering page on the physician's local computer.

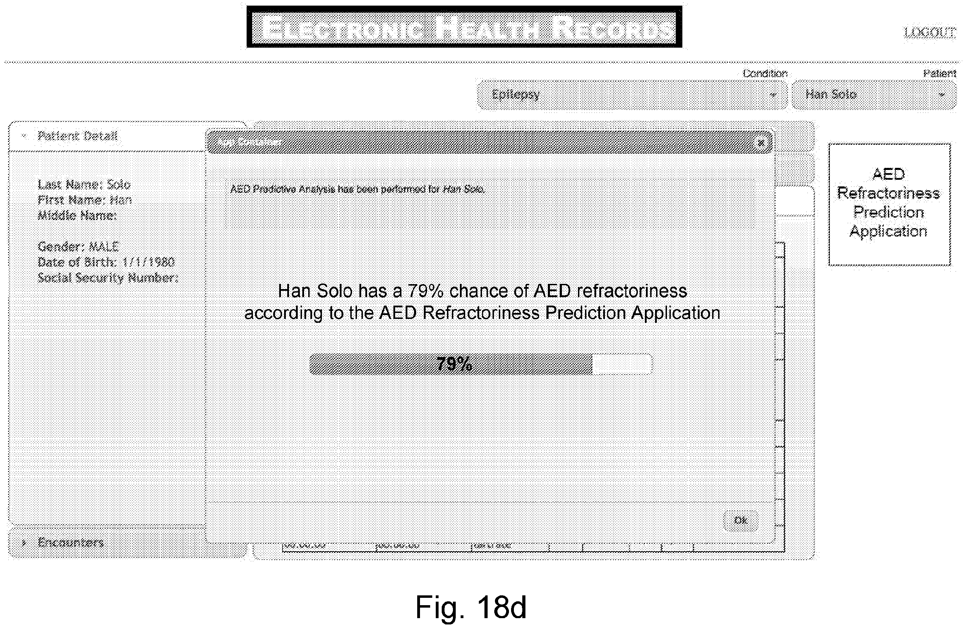

Next, in a step 216, the constructed feature set created by epilepsy feature mapping tool 132 with help of application service 124 are sent to predictive model deployment tool 126 for execution. When the execution of the predictive models is complete, the epilepsy refractoriness result of the patient is sent to application service 124 and rendered and provided to the user physician in the final report page 406 on the GUI, as shown in Fig. 18d. In one preferred embodiment, the refractoriness results of the patient is presented as a percentage likelihood that the patient will become refractory. Additional features of the report can be implemented on the physician's local computer in JavaScript as a client-side service.

As noted above, in some preferred embodiments, the predictive model is a recurrent neural network including a plurality of layers. One of the layers is an input layer providing the features as a one-hot or multi-hot vector in natural processing language, while another of the layers is an embedding layer receiving the one-hot or multi-hot vector from the input layer. The embedding layer includes a matrix grouping relevant events from the input layer to reduce the dimensions of the features at least fifty fold and in one preferred embodiment the embedding layer is pretrained via a Med2Vec technique. The recurrent neural network also includes at least one hidden layer including a plurality of recurrent neural network units and a classifier configured to classify each patient as refractory or non-refractory.

In the preceding specification, the invention has been described with reference to specific exemplary embodiments and examples thereof.

## Claims

1. A method of building an epilepsy refractoriness prediction model for predicting refractoriness of epilepsy patients comprising:
a) providing electronic health records data;
b) constructing a patient cohort from the electronic health records data by selecting patients based on failure of at least one anti-epilepsy drug;
c) constructing a set of features found in or derived from the electronic health records data,
wherein said features include:
(i) demographic features representing basic demographics of the patient such as age, gender and the geographic information of the patient;
(ii) comorbidity features representing features corresponding to the different comorbidities associated with epilepsy such as migraine, sleep related disorders, disorders and different kinds of mental disorders;
(iii) ecosystem and policy features representing factors which affect the care given to patients such as characteristics of the physicians treating the patients;
(iv) epilepsy status features representative of the status of epilepsy of patients, including details about patient encounters;
(v) treatment features representative of to the treatment regimen and medical procedures undertaken by patients in the observation period;
d) electronically processing the patient cohort to identify a subset of the features that are predictive for refractoriness for inclusion in a predictive model configured for classifying patients as refractory or non-refractory, including performing a statistical test on the features to identify which of the features have a statistical significance value within a predetermined range; and
e) training the predictive computerized model to classify the patients having at least one anti-epilepsy drug failure based on likelihood of becoming refractory.

2. The method as recited in claim 1 wherein the predictive computerized model is a recurrent neural network including a plurality of layers.

3. The method as recited in claim 2 wherein the recurrent neural network includes an input layer providing the features as a one-hot or multi-hot vector in natural processing language.

4. The method as recited in claim 3 wherein the recurrent neural network includes at least one hidden layer including a plurality of recurrent neural network units.

5. The method as recited in claim 3 wherein the recurrent neural network includes a classifier configured to classify each patient as refractory or non-refractory.

6. A computer platform for generating epilepsy refractoriness predictions comprising:
a) a client configured for interfacing with a data interface server, the data interface server configured to request formatted electronic medical records data for a patient from an electronic medical records database;
b) a feature mapping tool configured for mapping features from the formatted electronic medical records data into a further format,
wherein said feature mapping tool is configured for representing at least some of the features in the data as events each associated with a timestamp reflecting a temporal order in the patient's electronic medical records data to map the features from the formatted electronic medical records data into the further format, and
wherein the mapped features include demographic features, comorbidity features, ecosystem and policy features, epilepsy status features and treatment features.;
c) a model deployment tool configured for deploying a pretrained epilepsy refractoriness prediction model built using the method of claim 1; and
d) an epilepsy refractoriness prediction generator configured for generating an epilepsy refractoriness prediction for the patient by running the mapped features through the pretrained epilepsy refractoriness prediction model,
the epilepsy refractoriness prediction generator including an epilepsy refractoriness prediction application configured for generating a display representing the epilepsy refractoriness prediction.

7. The computer platform as recited in claim 6 wherein the epilepsy refractoriness prediction generator is configured for generating a graphical user interface for receiving an input from a user, the input being configured for generating a request for the patient's formatted electronic medical records data.

8. The computer platform as recited in claim 7 wherein epilepsy refractoriness prediction generator includes a backend service for generating the graphical user interface.

9. The computer platform as recited in claim 6 wherein the computer platform is configured to, upon being launched, access an authentication and authorization server securing the electronic medical records database and generate a prompt requiring the user to authenticate and authorize the computer platform to access the electronic medical records database.

10. The computer platform as recited in claim 6 wherein the pretrained epilepsy refractoriness prediction model is a recurrent neural network including a plurality of layers.

11. The computer platform as recited in claim 10 wherein the recurrent neural network includes an input layer providing the features as a one-hot or multi-hot vector in natural processing language.

12. The computer platform as recited in claim 11 wherein the recurrent neural network includes at least one hidden layer including a plurality of recurrent neural network units.

13. The computer platform as recited in claim 11 wherein the recurrent neural network includes a classifier configured to classify each patient as refractory or non-refractory.

14. A computerized method for generating epilepsy refractoriness predictions comprising:
a) providing a pretrained epilepsy refractoriness prediction model built using the method of claim 1;
b) requesting, via a client, formatted electronic medical records data for a patient from an electronic medical records database;
c) mapping features from the formatted electronic medical records data into a further format, wherein the mapping the features includes representing at least some of the features in the data as events each associated with a timestamp reflecting a temporal order in the patient's electronic medical records data to map the features from the formatted electronic medical records data into the further format, and wherein the mapped features include demographic features, comorbidity features, ecosystem and policy features, epilepsy status features and treatment features;
d) generating an epilepsy refractoriness prediction for the patient by running the mapped features through the pretrained epilepsy refractoriness prediction model; and
e) generating a display representing the epilepsy refractoriness prediction.

15. The method as recited in claim 14 further comprising generating a graphical user interface for receiving an input from a user, the input being configured for generating a request for the patient's formatted electronic medical records data.

16. The method as recited in claim 14 further comprising accessing an authentication and authorization server securing the electronic medical records database and generating a prompt requiring the user to authenticate and authorize the epilepsy refractoriness prediction application to access the electronic medical records database.

## Patentansprüche

1. Verfahren zum Aufbau eines Epilepsie-Refraktäritätsvorhersagemodells zur Vorhersage von Refraktärität von Epilepsiepatienten, umfassend:
a) Bereitstellen von Daten aus elektronischen Gesundheitsakten;
b) Erstellen einer Patientenkohorte aus den Daten aus elektronischen Gesundheitsakten durch Auswählen von Patienten auf der Grundlage des Versagens mindestens eines Antiepilepsie-Medikaments;
c) Erstellen eines Satzes von Merkmalen, die in den Daten aus elektronischen Gesundheitsakten gefunden oder von diesen abgeleitet wurden, wobei die Merkmale beinhalten:
(i) demografische Merkmale, die grundlegende demografische Daten des Patienten wie etwa Alter, Geschlecht und geografische Informationen des Patienten repräsentieren;
(ii) Komorbiditätsmerkmale, die Merkmale repräsentieren, die den verschiedenen Komorbiditäten im Zusammenhang mit Epilepsie entsprechen, wie etwa Migräne, schlafbezogene Störungen, Erkrankungen und verschiedene Arten von psychischen Störungen;
(iii) Gesundheitssystem- und Versicherungsmerkmale, die Faktoren repräsentieren, die sich auf die Versorgung der Patienten auswirken, wie etwa Eigenschaften der behandelnden Ärzte;
(iv) Epilepsie-Statusmerkmale, die für den Status der Epilepsie von Patienten repräsentativ sind, einschließlich Einzelheiten zu Patientenbegegnungen;
(v) Behandlungsmerkmale, die repräsentativ für das Behandlungsschema und die medizinischen Verfahren sind, die von den Patienten während des Beobachtungszeitraums durchgeführt wurden;
d) elektronisches Verarbeiten der Patientenkohorte zum Identifizieren einer Untergruppe der Merkmale, die prädiktiv für die Refraktärität sind, zu dem Zweck, sie in ein Vorhersagemodell aufzunehmen, das zur Klassifizierung von Patienten als refraktär oder nicht-refraktär ausgelegt ist, einschließlich des Durchführens eines statistischen Tests an den Merkmalen zum Identifizieren, welche der Merkmale einen statistischen Signifikanzwert innerhalb eines vorbestimmten Bereichs haben; und
e) Trainieren des computergestützten Vorhersagemodells zum Klassifizieren der Patienten mit mindestens einem Versagen eines Antiepilepsie-Medikaments auf der Grundlage der Wahrscheinlichkeit, refraktär zu werden.

2. Verfahren nach Anspruch 1, wobei das computergestützte Vorhersagemodell ein rekurrentes neuronales Netz einschließlich einer Vielzahl von Schichten ist.

3. Verfahren nach Anspruch 2, wobei das rekurrente neuronale Netz eine Eingabeschicht beinhaltet, die die Merkmale als One-Hot- oder Multi-Hot-Vektor in natürlicher Verarbeitungssprache bereitstellt.

4. Verfahren nach Anspruch 3, wobei das rekurrente neuronale Netz mindestens eine versteckte Schicht einschließlich einer Vielzahl von rekurrenten neuronalen Netzeinheiten beinhaltet.

5. Verfahren nach Anspruch 3, wobei das rekurrente neuronale Netz einen Klassifikator beinhaltet, der dafür ausgelegt ist, jeden Patienten als refraktär oder nicht-refraktär zu klassifizieren.

6. Computerplattform zum Erzeugen von Epilepsie-Refraktäritätsvorhersagen, umfassend:
a) einen Client, der für die Verbindung mit einem Datenschnittstellenserver ausgelegt ist, wobei der Datenschnittstellenserver dafür ausgelegt ist, formatierte Daten aus elektronischen Gesundheitsakten für einen Patienten von einer Datenbank für elektronische Gesundheitsakten anzufordern;
b) ein Merkmalsabbildungswerkzeug, das dafür ausgelegt ist, Merkmale aus den formatierten Daten aus elektronischen Gesundheitsakten in ein weiteres Format abzubilden,
wobei das Merkmalsabbildungswerkzeug dafür ausgelegt ist, mindestens einige der Merkmale in den Daten als Ereignisse zu repräsentieren, die jeweils einem Zeitstempel zugeordnet sind, der eine zeitliche Reihenfolge in den Daten aus elektronischen Gesundheitsakten des Patienten widerspiegelt, zu dem Zweck, die Merkmale aus den formatierten Daten aus elektronischen Gesundheitsakten in das weitere Format abzubilden, und
wobei die abgebildeten Merkmale demografische Merkmale, Komorbiditätsmerkmale, Gesundheitssystem- und Versicherungsmerkmale, Epilepsiestatusmerkmale und Behandlungsmerkmale beinhalten;
c) ein Modellbereitstellungswerkzeug, das dafür ausgelegt ist, ein vortrainiertes Epilepsie-Refraktäritätsvorhersagemodell bereitzustellen, das unter Verwendung des Verfahrens nach Anspruch 1 aufgebaut wurde; und
d) einen Epilepsie-Refraktäritätsvorhersagegenerator, der dafür ausgelegt ist, eine Epilepsie-Refraktäritätsvorhersage für den Patienten zu erzeugen, indem er die abgebildeten Merkmale durch das vortrainierte Epilepsie-Refraktäritätsvorhersagemodell verarbeiten lässt,
wobei der Epilepsie-Refraktäritätsvorhersagegenerator eine Epilepsie-Refraktäritätsvorhersageanwendung beinhaltet, die dafür ausgelegt ist, eine Anzeige zu erzeugen, die die Epilepsie-Refraktäritätsvorhersage repräsentiert.

7. Computerplattform nach Anspruch 6, wobei der Epilepsie-Refraktäritätsvorhersagegenerator dafür ausgelegt ist, eine grafische Benutzerschnittstelle zum Empfangen einer Eingabe von einem Benutzer zu erzeugen, wobei die Eingabe dafür ausgelegt ist, eine Anforderung für die formatierten Daten aus elektronischen Gesundheitsakten des Patienten zu erzeugen.

8. Computerplattform nach Anspruch 7, wobei der Epilepsie-Refraktäritätsvorhersagegenerator einen Backend-Dienst zum Erzeugen der grafischen Benutzerschnittstelle beinhaltet.

9. Computerplattform nach Anspruch 6, wobei die Computerplattform dafür ausgelegt ist, nach dem Start auf einen Authentifizierungs- und Autorisierungsserver zuzugreifen, der die Datenbank für elektronische Gesundheitsakten sichert, und eine Aufforderung zu erzeugen, die den Benutzer auffordert, die Computerplattform zum Zugriff auf die Datenbank für elektronische Gesundheitsakten zu authentifizieren und zu autorisieren.

10. Computerplattform nach Anspruch 6, wobei das vortrainierte Epilepsie-Refraktäritätsvorhersagemodell ein rekurrentes neuronales Netz einschließlich einer Vielzahl von Schichten ist.

11. Computerplattform nach Anspruch 10, wobei das rekurrente neuronale Netz eine Eingabeschicht beinhaltet, die die Merkmale als One-Hot- oder Multi-Hot-Vektor in natürlicher Verarbeitungssprache bereitstellt.

12. Computerplattform nach Anspruch 11, wobei das rekurrente neuronale Netz mindestens eine versteckte Schicht einschließlich einer Vielzahl von rekurrenten neuronalen Netzeinheiten beinhaltet.

13. Computerplattform nach Anspruch 11, wobei das rekurrente neuronale Netz einen Klassifikator beinhaltet, der dafür ausgelegt ist, jeden Patienten als refraktär oder nicht-refraktär zu klassifizieren.

14. Computergestütztes Verfahren zum Erzeugen von Epilepsie-Refraktäritätsvorhersagen, umfassend:
a) Bereitstellen eines vortrainierten Epilepsie-Refraktäritätsvorhersagemodells, das unter Verwendung des Verfahrens nach Anspruch 1 aufgebaut wurde;
b) Anfordern, über einen Client, von formatierten Daten aus elektronischen Gesundheitsakten für einen Patienten aus einer Datenbank für elektronische Gesundheitsakten;
c) Abbilden von Merkmalen aus den formatierten Daten aus elektronischen Gesundheitsakten in ein weiteres Format, wobei das Abbilden der Merkmale das Repräsentieren mindestens einiger der Merkmale in den Daten als Ereignisse beinhaltet, die jeweils einem Zeitstempel zugeordnet sind, der eine zeitliche Reihenfolge in den Daten aus elektronischen Gesundheitsakten des Patienten widerspiegelt, zu dem Zweck, die Merkmale aus den formatierten Daten aus elektronischen Gesundheitsakten in das weitere Format abzubilden, und wobei die abgebildeten Merkmale demografische Merkmale, Komorbiditätsmerkmale, Gesundheitssystem- und Versicherungsmerkmale, Epilepsiestatusmerkmale und Behandlungsmerkmale beinhalten;
d) Erzeugen einer Epilepsie-Refraktäritätsvorhersage für den Patienten durch Verarbeiten der abgebildeten Merkmale durch das vortrainierte Epilepsie-Refraktäritätsvorhersagemodell; und
e) Erzeugen einer Anzeige, die die Epilepsie-Refraktäritätsvorhersage repräsentiert.

15. Verfahren nach Anspruch 14, ferner umfassend das Erzeugen einer grafischen Benutzerschnittstelle zum Empfangen einer Eingabe von einem Benutzer, wobei die Eingabe dafür ausgelegt ist, eine Anforderung für die formatierten Daten aus elektronischen Gesundheitsakten des Patienten zu erzeugen.

16. Verfahren nach Anspruch 14, ferner umfassend das Zugreifen auf einen Authentifizierungs- und Autorisierungsserver, der die Datenbank für elektronische Gesundheitsakten sichert, und das Erzeugen einer Aufforderung, die den Benutzer auffordert, die Epilepsie-Refraktäritätsvorhersageanwendung zum Zugriff auf die Datenbank für elektronische Gesundheitsakten zu authentifizieren und zu autorisieren.

## Revendications

1. Procédé d'élaboration d'un modèle de prédiction de réfractarité d'épilepsie permettant de prédire la réfractarité de patients épileptiques comprenant :
a) la fourniture de données de dossiers médicaux électroniques ;
b) la construction d'une cohorte de patients à partir des données de dossiers médicaux électroniques en sélectionnant des patients sur la base de l'échec d'au moins un médicament antiépileptique ;
c) la construction d'un ensemble de caractéristiques trouvées dans les données de dossiers médicaux électroniques ou dérivées de celles-ci,
dans lequel lesdites caractéristiques comportent :
(i) des caractéristiques sociodémographiques représentant des données sociodémographiques de base du patient telles que l'âge, le sexe et les informations géographiques du patient ;
(ii) des caractéristiques de comorbidité représentant des caractéristiques correspondant aux différentes comorbidités associées à l'épilepsie telles que la migraine, les troubles du sommeil, les troubles et différents types de troubles mentaux ;
(iii) des caractéristiques d'écosystème et de politique représentant des facteurs qui influent sur les soins prodigués aux patients, telles que des caractéristiques des médecins qui traitent les patients ;
(iv) des caractéristiques d'état d'épilepsie représentant l'état de l'épilepsie des patients, y compris des détails sur les rencontres avec les patients ;
(v) des caractéristiques de traitement représentant le régime thérapeutique et les actes médicaux suivis par les patients au cours de la période d'observation ;
d) le traitement électronique de la cohorte de patients afin d'identifier un sous-ensemble des caractéristiques qui prédisent la réfractarité pour une inclusion dans un modèle prédictif configuré pour classer les patients comme réfractaires ou non réfractaires, notamment en effectuant un test statistique sur les caractéristiques afin d'identifier celles dont la valeur de signification statistique se situe dans une plage prédéterminée ; et
e) l'apprentissage du modèle informatisé prédictif pour classer les patients présentant au moins un échec de médicament antiépileptique sur la base de la probabilité qu'ils deviennent réfractaires.

2. Procédé selon la revendication 1 dans lequel le modèle informatisé prédictif est un réseau neuronal récurrent comprenant une pluralité de couches.

3. Procédé selon la revendication 2 dans lequel le réseau neuronal récurrent comporte une couche d'entrée fournissant les caractéristiques sous la forme d'un vecteur « one-hot » ou « multi-hot » dans un langage de traitement naturel.

4. Procédé selon la revendication 3 dans lequel le réseau neuronal récurrent comporte au moins une couche cachée comprenant une pluralité d'unités de réseau neuronal récurrent.

5. Procédé selon la revendication 3 dans lequel le réseau neuronal récurrent comporte un classificateur configuré pour classer chaque patient comme réfractaire ou non réfractaire.

6. Plate-forme informatique permettant de générer des prédictions de réfractarité d'épilepsie comprenant :
a) un client configuré pour établir l'interface avec un serveur d'interface de données, le serveur d'interface de données étant configuré pour demander des données de dossiers médicaux électroniques formatées pour un patient à partir d'une base de données de dossiers médicaux électroniques ;
b) un outil de mise en correspondance de caractéristiques configuré pour mettre en correspondance des caractéristiques des données de dossiers médicaux électroniques formatées dans un autre format,
dans laquelle ledit outil de mise en correspondance de caractéristiques est configuré pour représenter au moins certaines des caractéristiques des données sous forme d'événements associés chacun à un horodatage reflétant un ordre temporel dans les données du dossier médical électronique du patient afin de mettre en correspondance les caractéristiques des données de dossier médical électronique formatées dans l'autre format, et
dans laquelle les caractéristiques mises en correspondance comportent des caractéristiques sociodémographiques, des caractéristiques de comorbidité, des caractéristiques d'écosystème et de politique, des caractéristiques d'état d'épilepsie et des caractéristiques de traitement ;
c) un outil de déploiement de modèle configuré pour déployer un modèle de prédiction de réfractarité d'épilepsie préalablement entraîné élaboré en utilisant le procédé selon la revendication 1 ; et
d) un générateur de prédiction de réfractarité d'épilepsie configuré pour générer une prédiction de réfractarité d'épilepsie pour le patient en exécutant les caractéristiques mise en correspondance dans le modèle de prédiction de réfractarité d'épilepsie préalablement entraîné,
le générateur de prédiction de réfractarité d'épilepsie comportant une application de prédiction de réfractarité d'épilepsie configurée pour générer un affichage représentant la prédiction de réfractarité d'épilepsie.

7. Plate-forme informatique selon la revendication 6 dans laquelle le générateur de prédiction de réfractarité d'épilepsie est configuré pour générer une interface utilisateur graphique pour recevoir une entrée d'un utilisateur, l'entrée étant configurée pour générer une demande pour les données de dossier médical électronique formatées du patient.

8. Plate-forme informatique selon la revendication 7 dans laquelle le générateur de prédiction de réfractarité d'épilepsie comporte un service de fond pour générer l'interface utilisateur graphique.

9. Plate-forme informatique selon la revendication 6, la plate-forme informatique étant configurée pour, lors de son lancement, accéder à un serveur d'authentification et d'autorisation sécurisant la base de données de dossiers médicaux électroniques et générer une invite demandant à l'utilisateur de s'authentifier et d'autoriser la plate-forme informatique à accéder à la base de données de dossiers médicaux électroniques.

10. Plate-forme informatique selon la revendication 6 dans laquelle le modèle de prédiction de réfractarité d'épilepsie préalablement entraîné est un réseau neuronal récurrent comportant une pluralité de couches.

11. Plate-forme informatique selon la revendication 10 dans laquelle le réseau neuronal récurrent comporte une couche d'entrée fournissant les caractéristiques sous la forme d'un vecteur « one-hot » ou « multi-hot » dans un langage de traitement naturel.

12. Plate-forme informatique selon la revendication 11 dans laquelle le réseau neuronal récurrent comporte au moins une couche cachée comportant une pluralité d'unités de réseau neuronal récurrent.

13. Plate-forme informatique selon la revendication 11 dans laquelle le réseau neuronal récurrent comporte un classificateur configuré pour classer chaque patient comme réfractaire ou non réfractaire.

14. Procédé informatisé permettant de générer des prédictions de réfractarité d'épilepsie comprenant :
a) la fourniture d'un modèle de prédiction de réfractarité d'épilepsie préalablement entraîné élaboré en utilisant le procédé selon la revendication 1 ;
b) la demande, par le biais d'un client, de données de dossiers médicaux électroniques formatées pour un patient à partir d'une base de données de dossiers médicaux électroniques ;
c) la mise en correspondance de caractéristiques à partir des données de dossiers médicaux électroniques formatées dans un autre format, la mise en correspondance des caractéristiques comportant la représentation d'au moins certaines des caractéristiques des données en tant qu'événements associés chacun à un horodatage reflétant un ordre temporel dans les données de dossier médical électronique du patient pour mettre en correspondance les caractéristiques des données de dossiers médicaux électroniques formatées dans l'autre format, et dans lequel les caractéristiques mises en correspondance comportent des caractéristiques sociodémographiques, des caractéristiques de comorbidité, des caractéristiques d'écosystème et de politique, des caractéristiques d'état de l'épilepsie et des caractéristiques de traitement ;
d) la génération d'une prédiction de réfractarité d'épilepsie pour le patient en exécutant les caractéristiques mises en correspondance dans le modèle de prédiction de réfractarité d'épilepsie préalablement entraîné ; et
e) la génération d'un affichage représentant la prédiction de réfractarité d'épilepsie.

15. Procédé selon la revendication 14 comprenant en outre la génération d'une interface utilisateur graphique pour recevoir une entrée d'un utilisateur, l'entrée étant configurée pour générer une demande pour les données du dossier médical électronique formatées du patient.

16. Procédé selon la revendication 14 comprenant en outre l'accès à un serveur d'authentification et d'autorisation sécurisant la base de données de dossiers médicaux électroniques et la génération d'une invite demandant à l'utilisateur de s'authentifier et d'autoriser l'application de prédiction de réfractarité d'épilepsie à accéder à la base de données de dossiers médicaux électroniques.
